# EUROPEAN PATENT SPECIFICATION

(11) **EP 0 560 890 B1**
(45) Date of publication and mention of the grant of the patent: **12.01.2005**
(21) Application number: 92901551.9
(22) Date of filing: 26.11.1991
(51) Int. Cl.: C12N 15/54, C12P 21/08, C12N 9/12

(54) **A NOVEL PROTEIN TYROSINE KINASE**
PROTEIN-TYROSINKINASE
NOUVELLE KINASE DE TYROSINE PROTEIQUE

(30) Priority: 28.11.1990 AU PK359490
(43) Date of publication of application: 22.09.1993
(60) Divisional application: 04019240.3
(73) Proprietor: LUDWIG INSTITUTE FOR CANCER RESEARCH, New York, New York 10158 (US)
(72) Inventor: WILKS, Andrew, Frederick, Doneaster East, VIC 3109 (AU); ZIEMIECKI, A., Inst. for Clinical and Experimental, CH-3004 Berne (CH); HARPUR, Ailsa, Mooroolbark, VIC 3138 (AU)
(74) Representative: Goldin, Douglas Michael
(86) International application number: PCT/US1991/008889
(87) International publication number: WO 1992/010519

(56) References cited:
- US-A- 4 543 439
- PROCEEDINGS OF THE NATIONAL ACADEMY OF SCIENCES OF USA vol. 86 , March 1989 , WASHINGTON US pages 1603 - 1607 A. F. WILKS 'Two putative Protein-Tyrosine Kinases identified by application of the polymerase chain reaction'
- ONCOGENE vol. 5, no. 9 , September 1990 pages 1329 - 1336 I. FIRMBACH-KRAFT ET AL 'TYK2, prototype of a novel class of non-receptor Tyrosine Kinase genes'
- MOLECULAR AND CELLULAR BIOLOGY vol. 11, no. 4 , April 1991 , WASHINGTON,US pages 2057 - 2065 A. F. WILKS ET AL 'Two novel Protein-Tyrosine Kinases, each with a second phosphotransferase-related catalytic domain, define a new class of Protein Kinase'
- Science, Volume 241, issued 01 July 1988 S.K. HANKS et al., "The Protein Kinase Family: Conserved Features and Decuced Phylogeny of the Catalytic Domains", pages 42-52, see entire document.
- Biochem. Biophys. Res. Comm., Volume 170, No. 1, issued 16 July 1990, D.C. GAUDETTE et al., "Effect of Genistein, A Tyrosine Kinase Inhibitor, on U46619-Induced Phosphorylation in Human Platelets", pages 238-242, see entire document.
- Science, Volume 252, issued 03 May 1991, C.A. KOCH et al., "SH2 and SH3 Domains: Elements that Control Interactions of Cytoplasmic Signaling Proteins", pages 668-674, see entire document.
- Mol. and Cell. Biol., Volume 6, No. 12, issued December 1986, I. SADOWSKI, et al, "A Noncatalytic Domain Conserved among Cytoplasmic Protein-Tyrosine Kinases Modifies the Kinase Function and Transforming Activity of Fujinami Sarcoma Virus P130gag-fpsn", pages 4396-4408, see entire document.
- Ann. Rev. Biochem., Volume 57, issued 1988, Y. YARDEN et al., "Growth Factor Receptor Tyrosine Kinases", pages 443-478, see entire document.

## Description

The present invention relates generally to a novel protein tyrosine kinase and to genetic sequences encoding same.

Protein tyrosine kinases (PTKs) are structurally well suited to a role intracellular signal transduction. Many growth factor receptors, for example, transduce the extracellular stimulus they receive through interaction with their cognate ligand via an intracellular tyrosine kinase domain. At least one of the non-receptor PTKs, namely LCK, is believed to mediate the transduction in T-cells of a signal from the interaction of a cell-surface protein (CD4) with a cross-linked anti-CD4 antibody.

The broader family of PTKs can be sub-divided on the basis of structural parameters of individual members. For example, the src family of PTKs now numbers 8 members (Marth et al., 1985; Nishizawa et al., 1986; Semba et al., 1986; Martinez et al., 1987; Sukegawa et al., 1987; Yamanishi et al., 1987; Hotzman et al., 1987; Dymecki et al., 1990), each with a characteristic complement of extra-catalytic domains, including an SH2, an SH3 domain and a variable ligand binding domain. It is clear that a process of gene duplication has taken place in this case, so that the evolutionarily successful thematic structure of this family can be employed in a variety of cellular contexts. Similar PTK structural sub-families exist based around the FGF receptor and the CSF-1 receptor (reviewed in Wilks, 1990).

However, one feature in common with the aforementioned PTKs is that each kinase bears a single highly related "catalytic" domain.

In accordance with the present invention a protein tyrosine kinase is provided which is distinct from those previously known. In particular, the protein tyrosine kinase of the present invention is unique since it possesses two protein kinase catalytic domains. Furthermore, the kinase does not bear an SH2 domain. The novel protein tyrosine kinase of the present invention represents a new subfamily or class of protein tyrosine kinase.

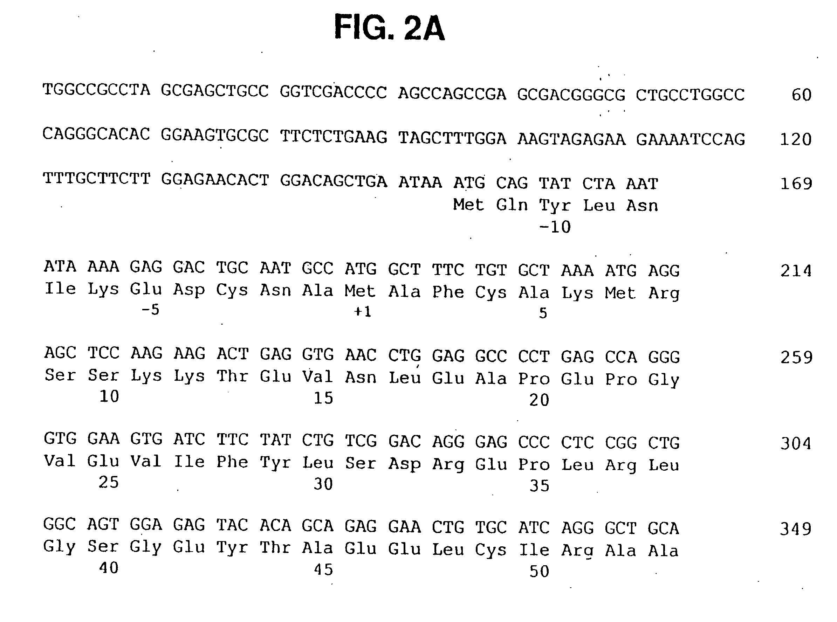

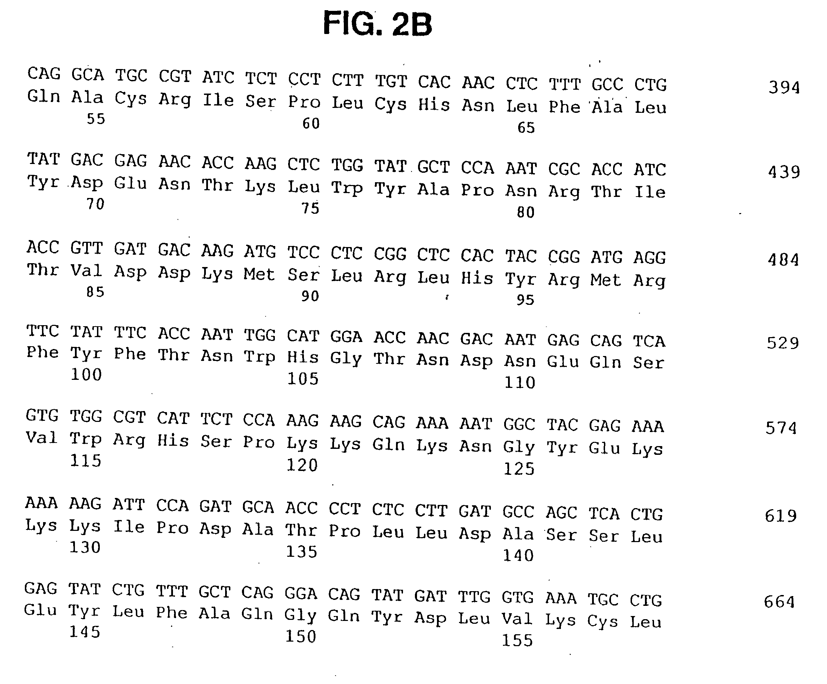

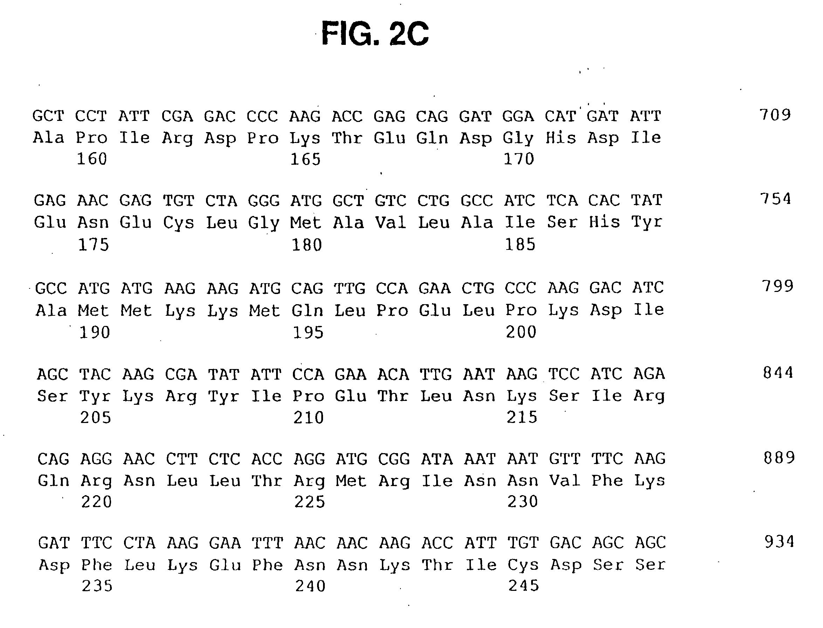

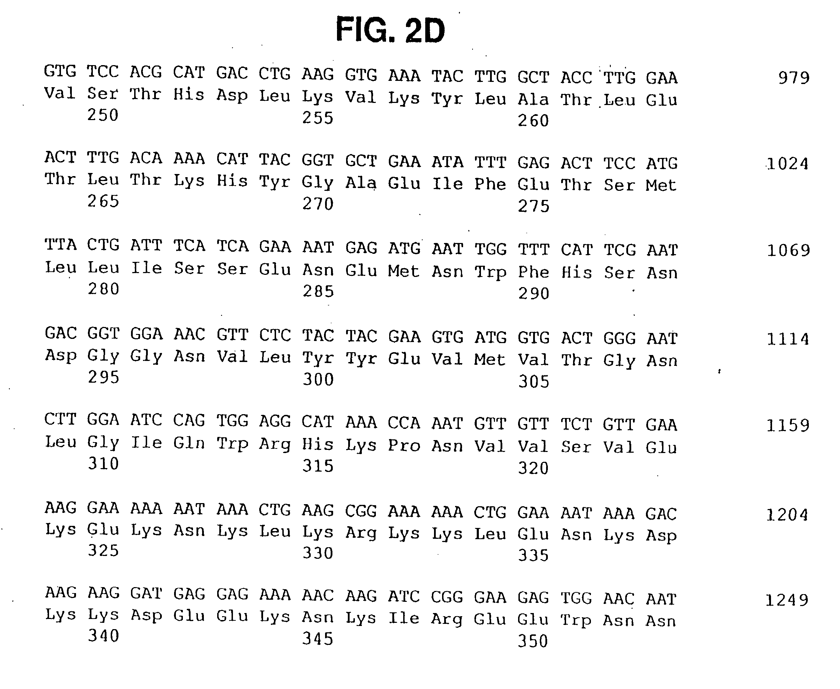

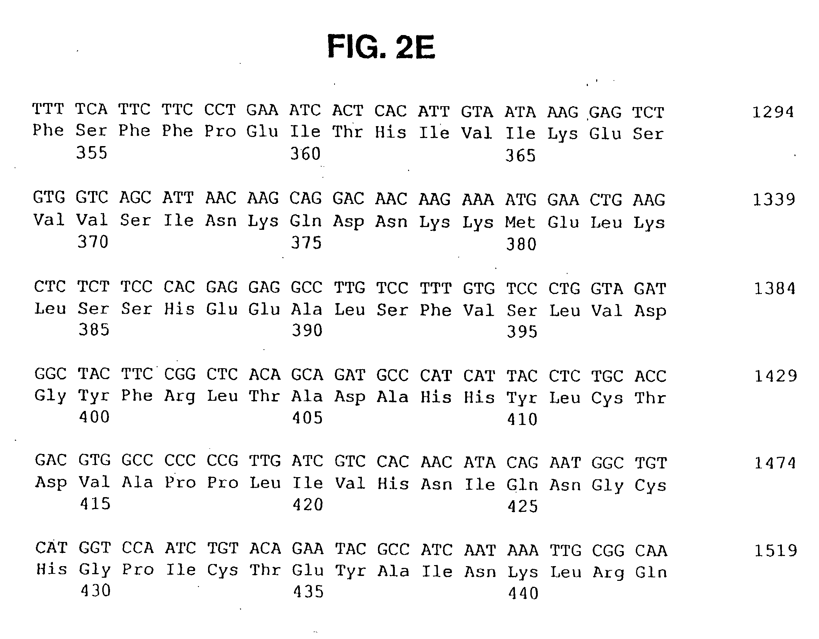

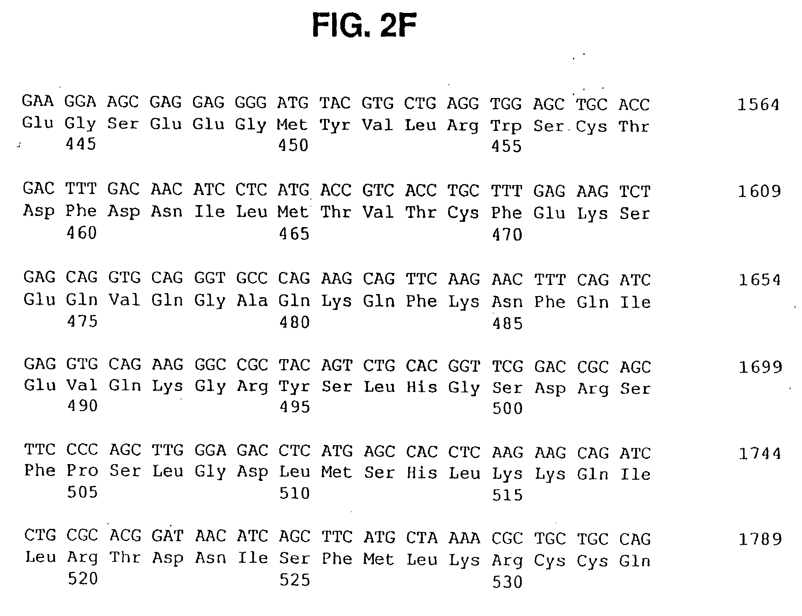

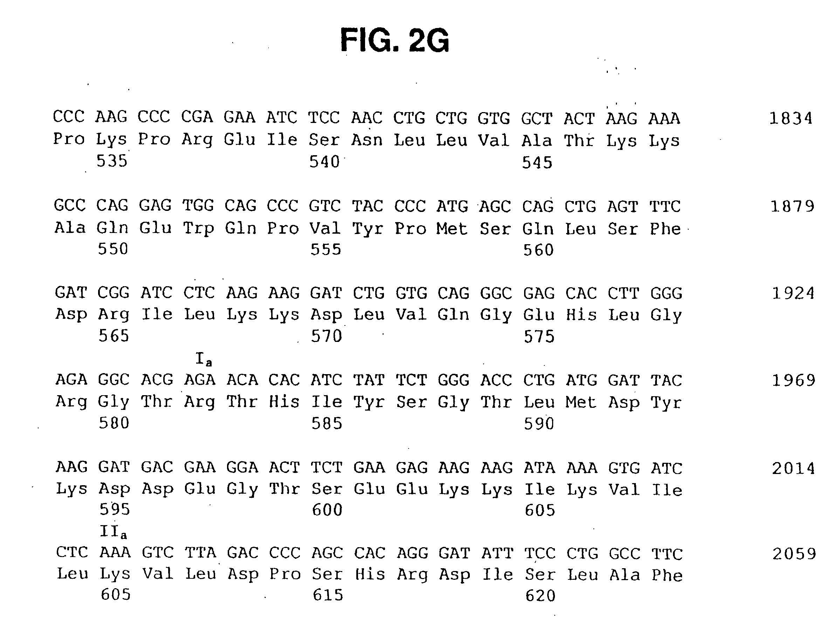

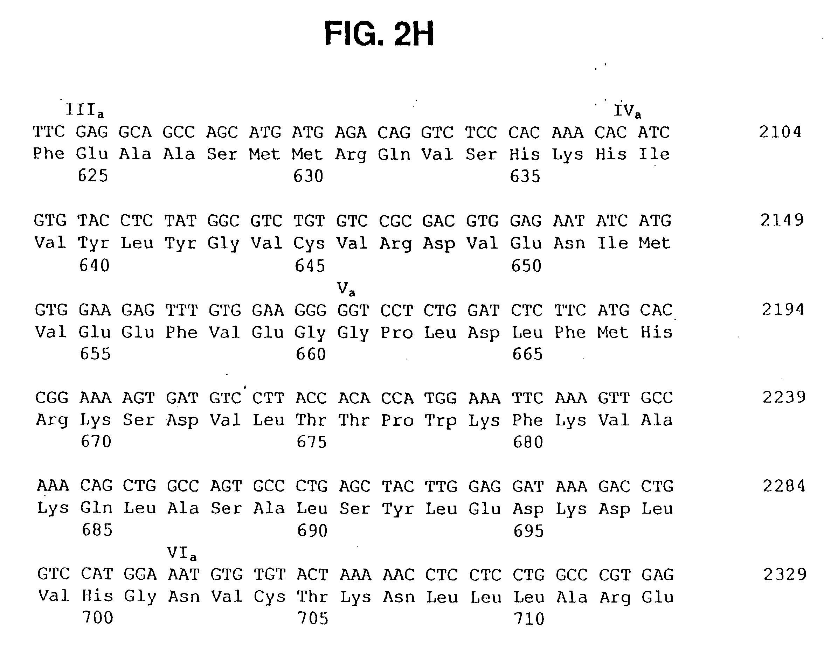

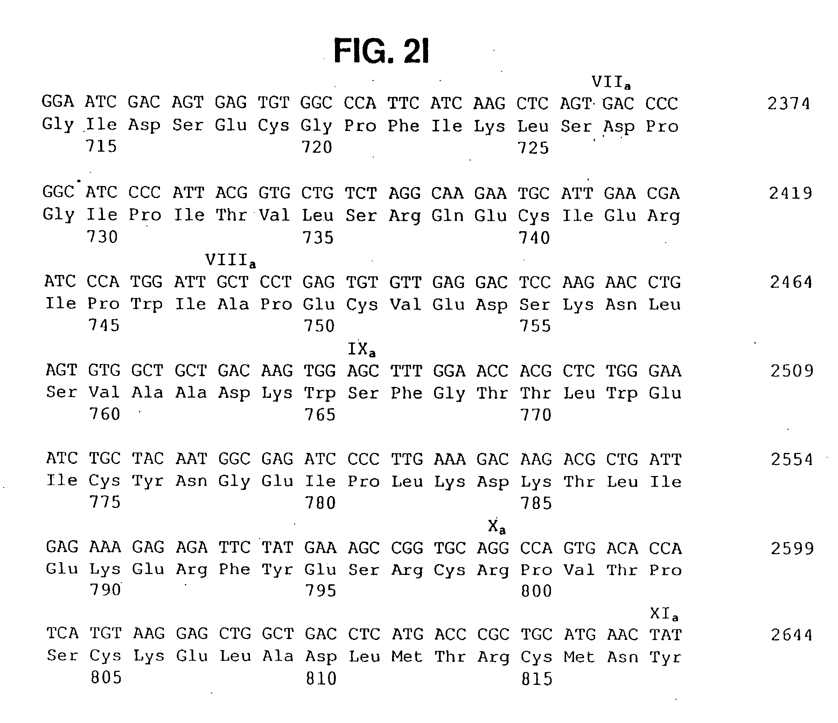

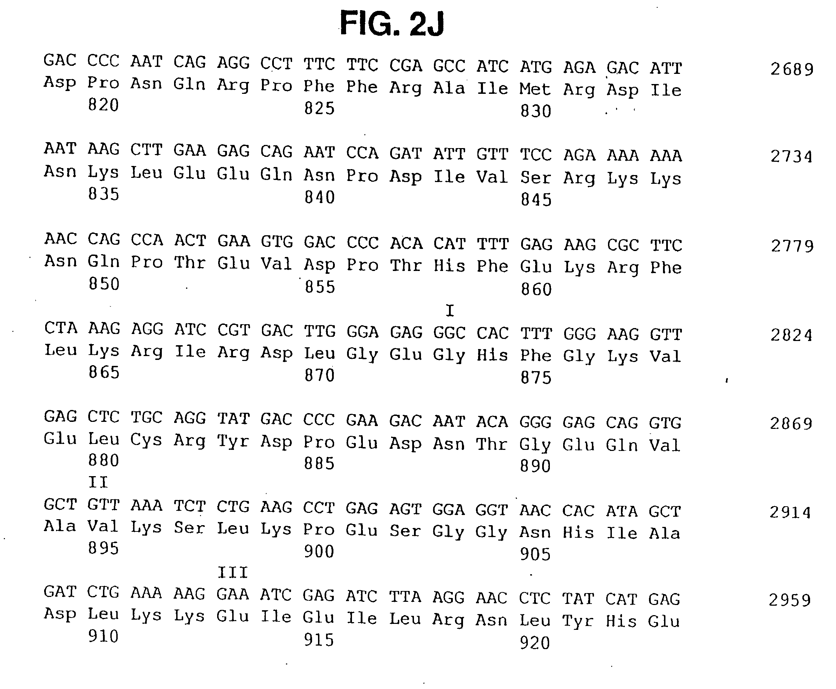

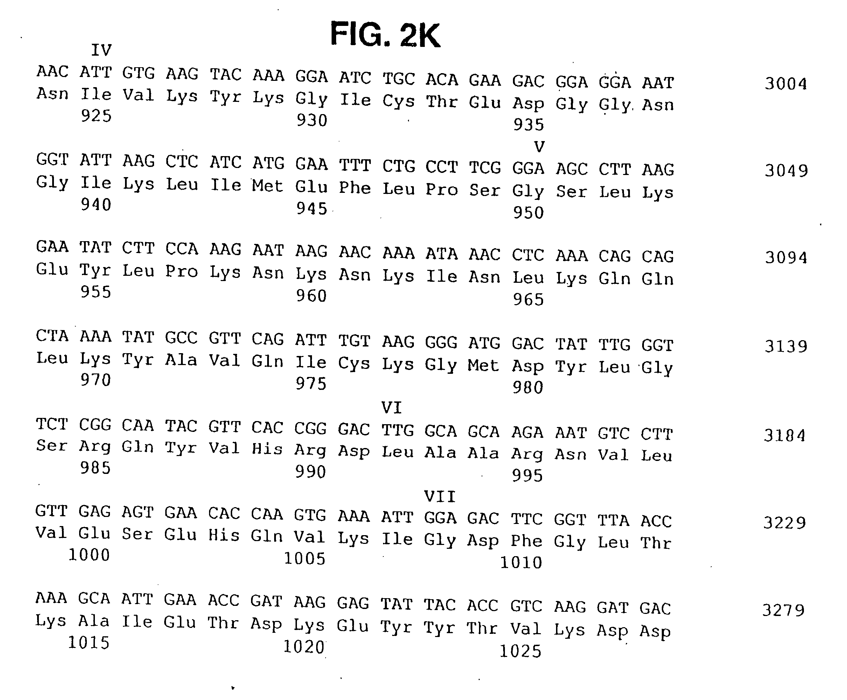

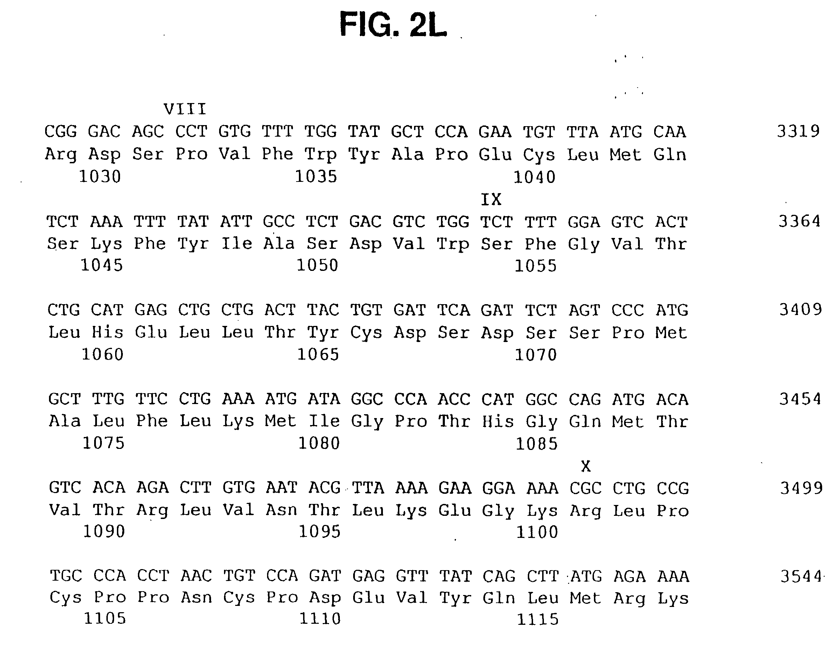

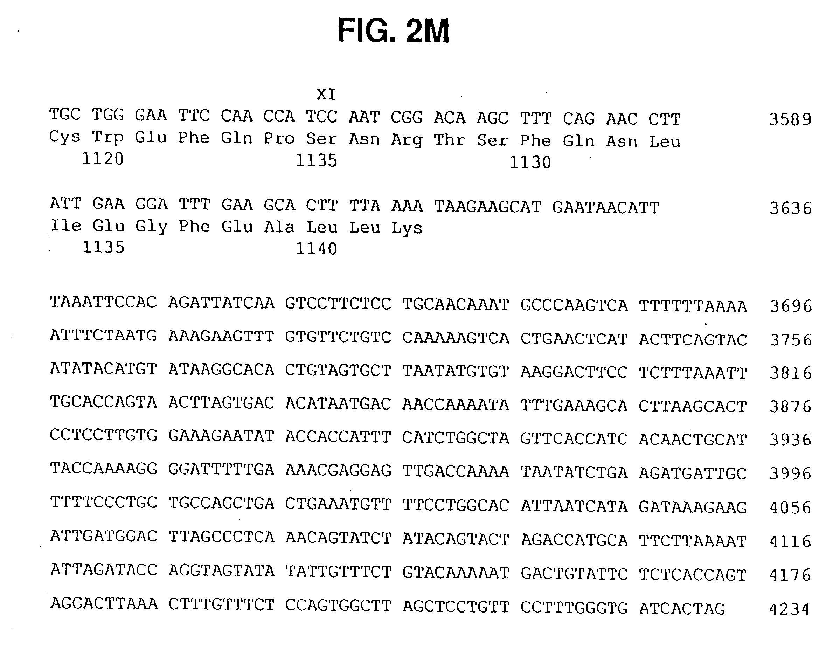

Accordingly, one aspect of the present invention provides a human or mouse protein tyrosine kinase-like (PTK) polypeptide comprising two protein kinase catalytic domains but no SH2 domain and (a) having the amino acid sequence of Figure 2 (JAK1) or (b) having an amino acid sequence which is encoded by a polynucleotide which hybridises to the complement of the polynucleotide sequence of Figure 2 under the stringency conditions of 6xSSC, 1% SDS, at 65°C and wash at 0.2xSSC, 0.1% SDS at 65°C.

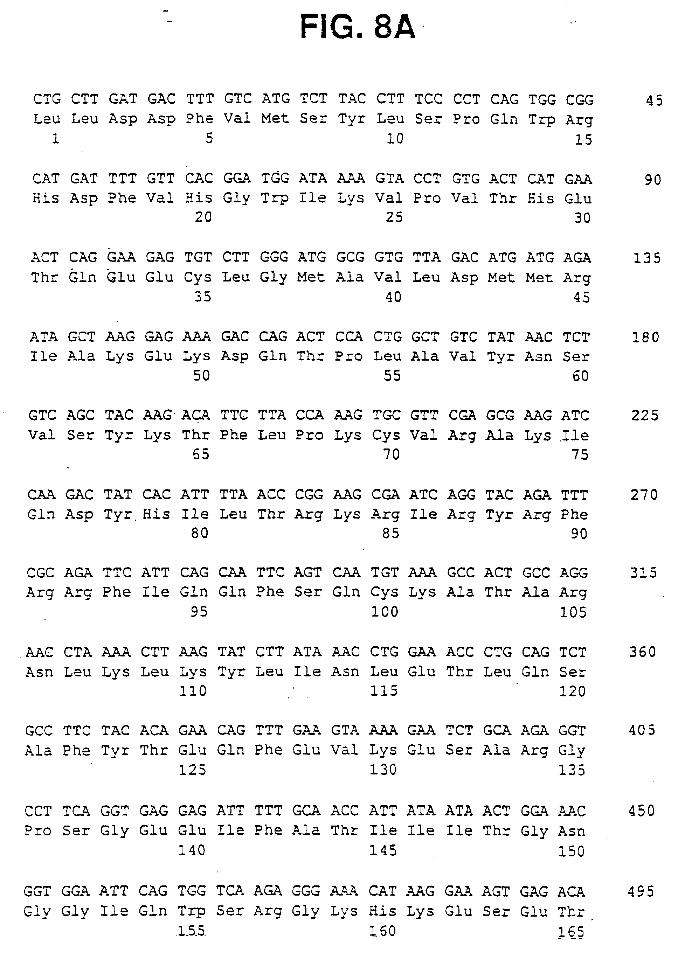

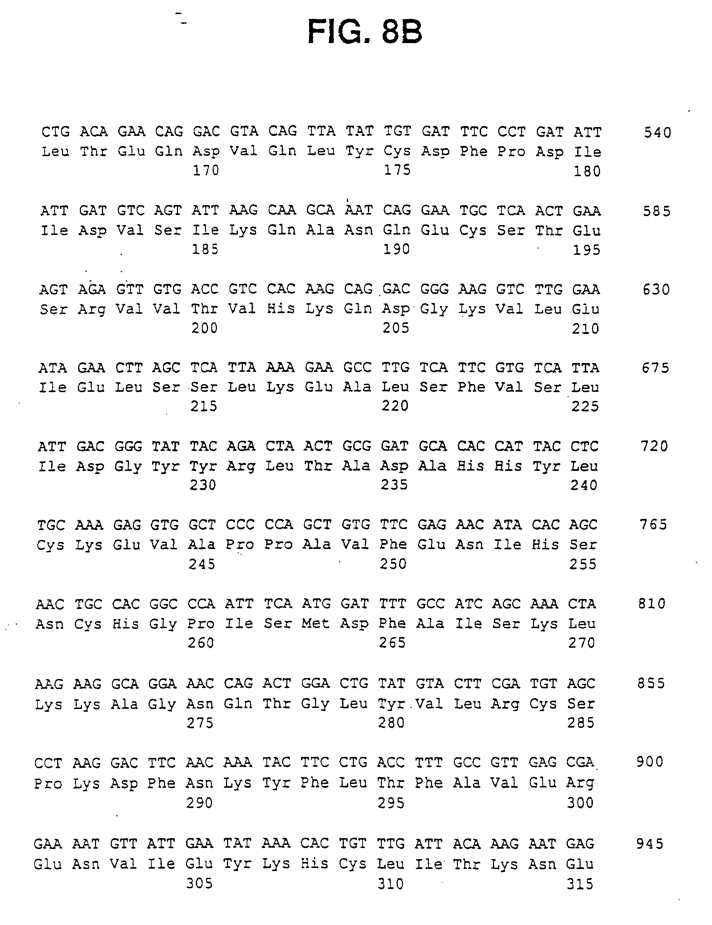

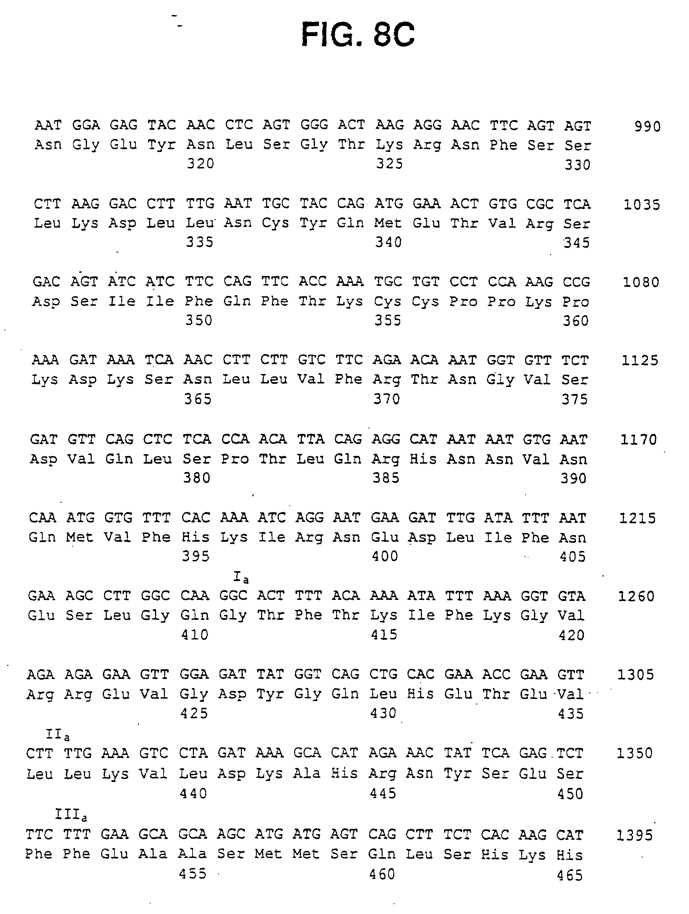

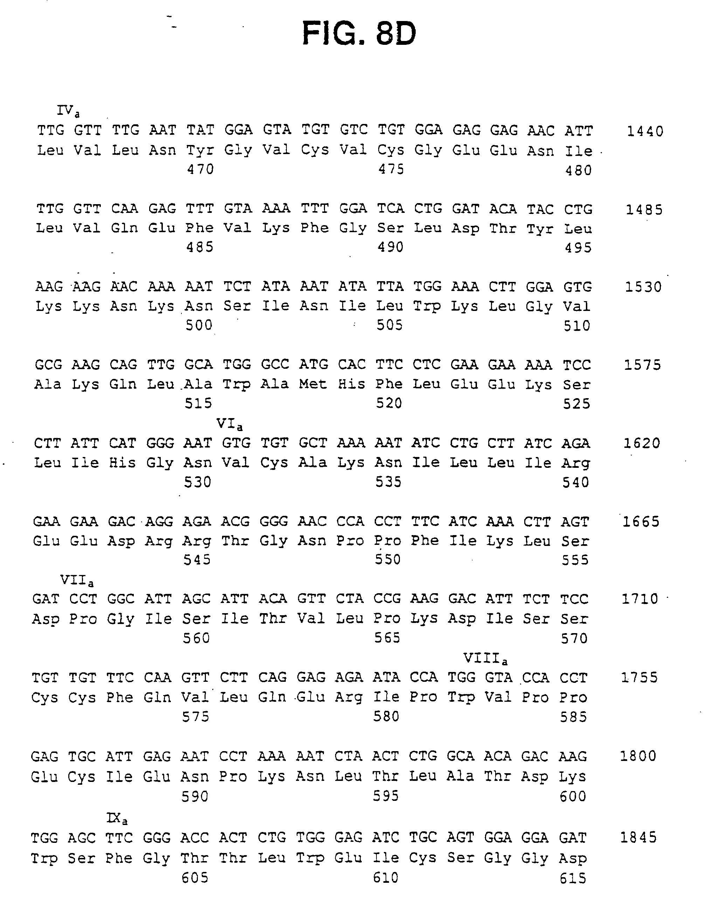

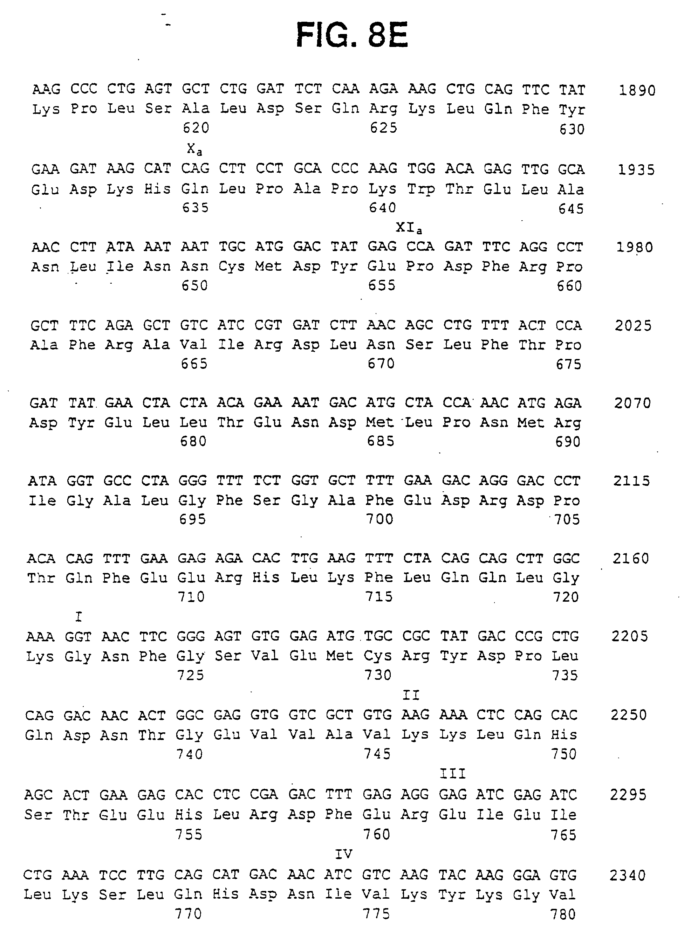

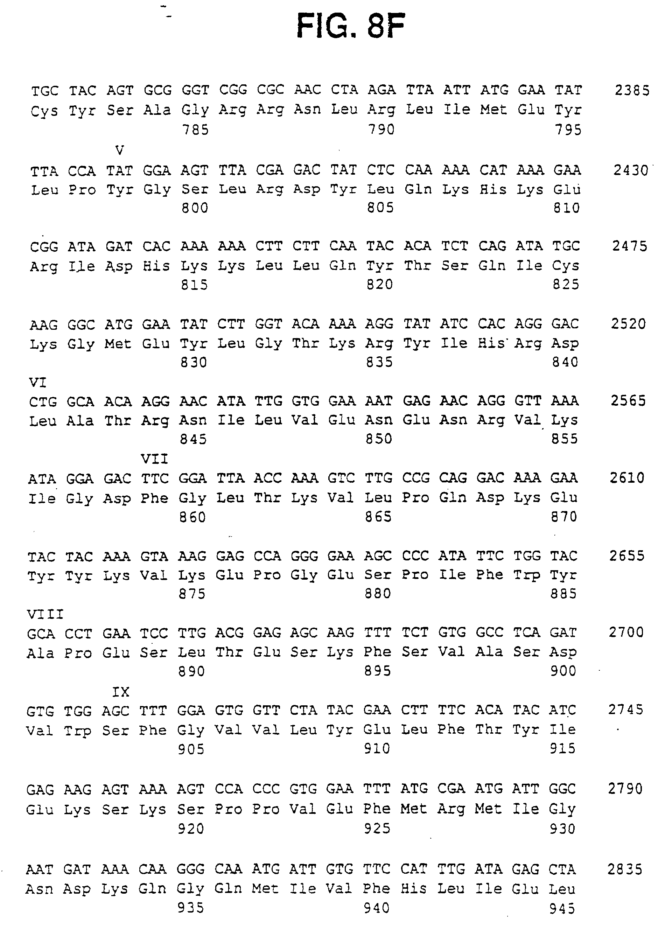

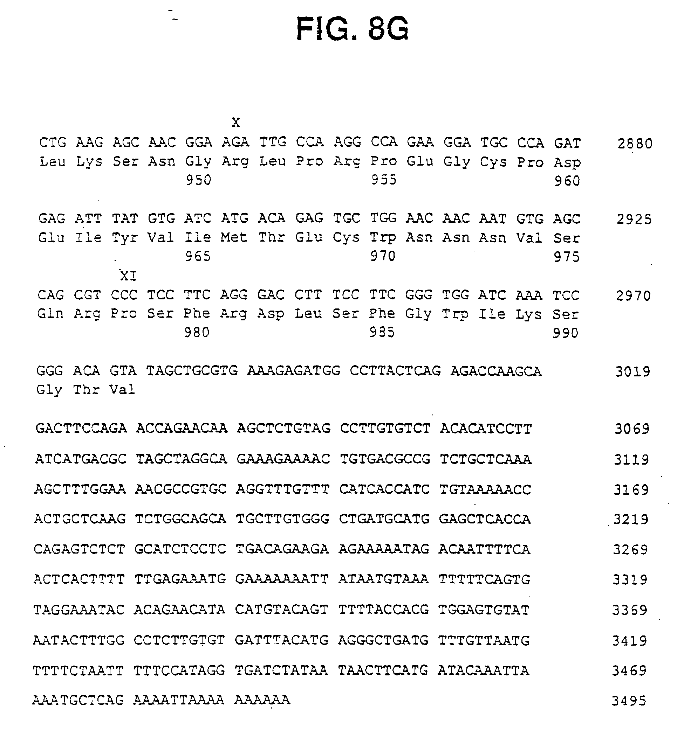

In another aspect there is provided a human or mouse protein tyrosine kinase-like (PTK) polypeptide comprising two protein kinase catalytic domains but no SH2 domain and (a) having the amino acid sequence of Figure 8 (JAK2) or (b) having an amino acid sequence which is encoded by a polynucleotide which hybridises to the complement of the polynucleotide sequence of Figure 8 under the stringency conditions of 6xSSC, 1% SDS, at 65°C and wash at 0.2xSSC, 0.1% SDS at 65°C.

Hereinafter, a protein having these characteristics will be referred to as a "JAK" (from Janus Kinase: Janus, in Encyclopaedia Britannica (11^{th} Ed) Vol XV pp 155-156). The present invention is specifically exemplified using JAK1 and JAK2 from humans and mice. We also describe extension to mutants, derivatives, analogues and homologues thereof. The term "protein tyrosine kinase-like molecule" (abbreviated herein to "PTK-like molecule") is used throughout the specification to encompass all members of the JAK family and to their mutants, derivatives, analogues and homologues.

In accordance with the present invention, there is provided a PTK-like molecule. Preferably the molecule is in biological pure or in substantially pure and/or synthetic form. The purity of the preparation is characterised by a sample comprising at least 70% by weight, preferably at least 80% by weight and most preferably at least 90% by weight PTK-like molecule. Alternatively, where the purity of the enzyme preparation is not critical, the present invention also encompasses an impure PTK-like molecule preparation but which possesses a substantial amount of JAK activity.

The present invention is directed to a naturally occurring PTK-like molecule, biologically pure or substantially pure as hereinbefore defined and to derivatives, functional analogues and homologues thereof. Such derivatives include polypeptides having single or multiple amino acid substitutions, deletions and/or additions relative to the naturally occurring sequence. These derivatives, functional analogues and homologues also encompass single or multiple substitutions, deletions and/or additions to any associated molecules such as carbohydrate, lipid and/or proteinacious moieties. Reference herein to "PTK-like molecules" includes all such derivatives, functional analogues and homologues. The present invention also extends to synthetic forms of the polypeptides which include recombinant molecules and molecules prepared by the stepwise addition of amino acids to groups of amino acids in defined order.

A range of derivatives and analogues of the PTK-like molecule are contemplated herein and include altering the molecule at its nucleotide sequence-encoding level, during its expression within a cell or in vitro or post-synthesis modification. Such derivatives and analogues include, but are not limited to, modifications to side chains, incorporation of unnatural amino acids during polypeptide synthesis and the use of crosslinkers and other methods which impose conformational constraints on the polypeptide or their analogues.

Examples of side chain modifications contemplated by the present invention include modifications of amino groups such as by reductive alkylation by reaction with an aldehyde followed by reduction with NaBH₄; amidination with methylacetimidate; acylation with acetic anhydride; carbamoylation of amino groups with cyanate; trinitrobenzylation of amino groups with 2, 4, 6, trinitrobenzene-sulphonic acid (TNBS); acylation of amino gropus with succinic anhydride and tetrahydrophthalic anhydride; and pyridoxylation of lysine with pyridoxal-5'-phosphate followed by reduction with NaBH₄.

The guanidino group of arginine residues may be modified by the formation of heterocyclic condensation products with reagents such as 2,3-butanedione, phenylglyoxal and glyoxal.

The carboxyl group may be modified by carbodiimide activation via O-acylisourea formtion followed by subsequent derivitisation, for example, to a corresponding amide.

Sulphydryl groups may be modified by methods such as carboxymethylation with iodoacetic acid or iodoacetamide; performic acid oxidation to cysteic acid; formation of a mixed disulphides with other thiol compounds; reaction with maleimide, maleic anhydride or other substituted maleimide; formation of mercurial derivatives using 4-chloromercuribenzoate, 4-chloromercuriphenylsulphonic acid, phenylmercury chloride, 2-chloromercuri-4-nitrophenol and other mercurials; carbamoylation with cyanate at alkaline pH.

Tryptophan residues may be modified by, for example, oxidation with N-bromosuccinimide or alkylation of the indole ring with 2-hydroxy-5-nitrobenzyl bromide or sulphenyl halides. Tyrosine residues on the other hand, may be altered by nitration with tetranitromethane to form a 3-nitrotyrosine derivative.

Modification of the imidazole ringe of a histidine residue may be accomplished by alkylation with iodoacetic acid derivatives or N-carbethoxylation with diethylpyrocarbonate.

Examples of incorporating unnatural amino acids and derivatives during polypeptide synthesis include, but are not limited to, use of norleucine, 4-amino butyric acid, 4-amino-3-hydroxy-5-phenlpentanoic acid, 6-aminohexanoic acid, t-butylglycine, norvaline, phenylglycine, ornithine, sarcosine, 4-amino-3-hydroxy-6-methylheptanoic acid, 2-thienyl alanine and/or D-isomers of amino acids.

Crosslinkers can be used, for example, to stabilise 3D conformations, using homo-bifunctional crosslinkers such as the bifunctional imido esters having (CH₂)ₙ spacer groups with n=1 to n = 6, glutaraldehyde, N-hydroxysuccinimide esters and hetero-bifunctional reagents which usually contain an amino-reactive moiety such as N-hydroxysuccinimide and another group specific-reactive moiety such as maleimido or dithio moiety (SH) or carbodiimide (COOH). In addition, polypeptides could be conformationally constrained by, for example, incorporation of C_{α} and N_{α}-methylamino acids, introduction of double bonds between C_{α} and C_{β} atoms of amino acids and the formation of cyclic polypeptides or analogues by introducing covalent bonds such as forming an amide bond between the N and C termini, between two side chains or between a side chain and the N or C terminus.

The present invention, therefore, extends to peptides or polypeptides and amino acid and/or chemical analogues thereof corresponding to regions of PTK-like molecules. Preferably, the PTK-like molecules will retain JAK activity. However, molecules carrying mutations in the catalytic domains rendering these inactive may be useful in, for example, titrating out activity and generation of antibodies such molecules are encompassed by the present invention.

The molecular weights of the PTK-like molecules of the present invention range from 100,000 to 200,000 daltons and preferably from 120,000 to 150,000 daltons.

In a most preferred embodiment, the present invention provides JAK1 and JAK2. JAK1 is an approximately 1142 amino acid molecule with a molecular weight of about 132,000 daltons and a nucleotide sequence shown in Figure 2. JAK2 is an approximately 1,100 amino acid molecule with a molecular weight of about 130,000 daltons and with a nucleotide sequence shown in Figure 8.

The present invention also provides a polypeptide comprising an amino acid sequence selected from residues 576 to 825 of Figure 2, residues 868 to 1130 of Figure 2, residues 715 to 980 of Figure 8 or residues 400 to 660 of Figure 8.

The present invention is also directed to genetic sequences including DNA, cDNA and mRNA which encode the PTK-like molecules hereindescribed. Such genetic sequences include single or multiple nucleotide substitutions, deletions and/or additions relative to the naturally occurring sequence and extend to sequences encoding the derivatives, functional analogues and homologues of the PTK-like molecules. The present invention also provides these genetic sequences in vector and expression vector systems either *in vitro* or in a biological system (i.e. eukaryotic or prokaryotic cells) transformed with such vectors or genetic sequences. In a most preferred embodiment the present invention provides cDNA encoding JAK1 and JAK2 as set forth in Figures 2 and 8, respectively. A range of mutants can be obtained using standard techniques such as oligonucleotide mutagenesis and chemical mutagenesis, and all such mutants and derivatives are encompassed by the present invention.

The present invention also provides antibodies to a PTK-like molecule of the invention. Such antibodies may be monoclonal or polyclonal.

The PTK-like molecules of the present invention have varying utility such as in the phosphorylation of proteins, incorporation of labels and in the design of analogues, antagonists and agonists of JAKs.

Accordingly, another aspect of the present invention contemplates a method for phosphorylating a protein *in vitro* comprising contacting said protein with a phosphorylating effective amount of a PTK-like molecule of the invention, for a time and under conditions sufficient for said first protein to be phosphorylated.

The invention also provides such a method, wherein the molecular weight of the PTK-line molecule of the invention is from about 100,000 to about 200,000 Daltons, preferably from about 120,000 to about 150,000 Daltons.

The present invention is further described by reference to the following nonlimiting Figures and Examples.

In the Figures:

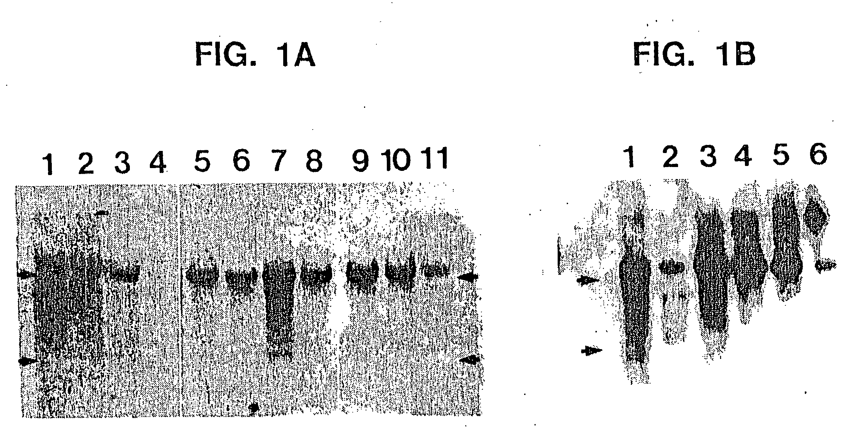

Figure 1 is a photographic representation of a Northern analysis of murine and human JAK1.
   A. 2µg aliquots of poly(A)+ mRNA from murine tissues: lane 1, lung: lane 2, liver: lane 3, kidney: lane 4, intestine: lane 5, brain: lane 6, skeletal muscle: lane 7, spleen: lane 8, salivary gland: lane 9, placenta: lane 10, mammary gland, were fractionated on a 1.0% agarose/formaldehyde (Moran et al, 1988) gel and the RNA transferred onto a Genescreen plus (Dupont) membrane. The transferred RNA was hybridized with a 1.8 kb ³²P-labelled murine JAK1 probe and the filter autoradiographed for 16 hr. at -70°C with two intensifying screens. The relative mobilities of 285 rRNA (upper arrow) and 185 rRNA (lower arrow) are shown.
   B. 2µg aliquots of poly(A)+ mRNA from the human haemopoietic cell lines: lane 1, HL60 (myelo-monocytic); lane 2, U937 (monocytic): lane 3, LK63 (pre-B): lane 4, RAJI (B-cell): lane 5, CEM (T-cell): lane 6, K562 (erythroleukaemia) were fractionated on a 1.0% agarose/formaldehyde (Moran et al, 1988) gel and the RNA transferred onto a Genescreen plus (Dupont) membrane. The transferred RNA was hybridized with a full-length ³²P-labelled human JAK1 probe and the filter autoradiographed for 16 hr. at -70°C with two intensifying screens. The relative mobilities of 28S rRNA (upper arrow) and 185 rRNA (lower arrow) are shown.

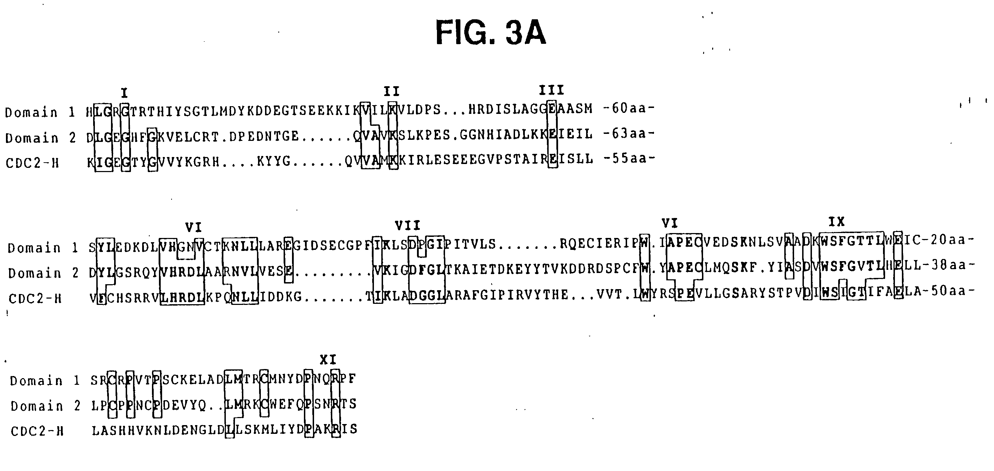

Figure 2 is a representation showing nucleotide sequence and predicted amino acid sequence of human JAK1. The DNA sequence is numbered at the end of each line of sequence from the first nucleotide of the largest clone (pHJ7,3), the amino acid sequence (in one letter code) is numbered from the putative AUG and appears above the line to which it refers. The two kinase catalytic domains are boxed with arrows, and kinase consensus motifs are enumerated according to the nomenclature of Hanks et al (1988). The suffix a (e.g. IIa) denotes the kinase related motifs present in the first kinase-related domain (designated domain- 1 in Fig.3a) also numbered according to the same nomenclature. The tyrosine residue in an analogous position to the autophosphorylation site of a number of other protein tyrosine kinases is marked with an inverted triangle. (Sequence Id. 1)

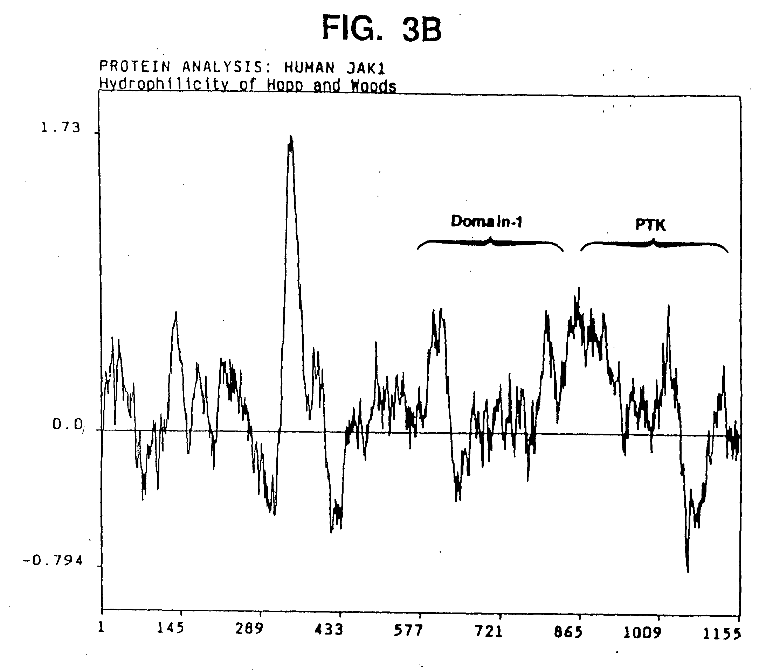

Figure 3 is a representation showing:
   Panel A. Amino-acid sequence comparison of the two kinase-related domains of JAK1. The amino-acid sequences (expressed in one-letter amino acid code) of the two kinase-related domains (domain- 1 amino-acids 576-825; domain-2 (PTK-domain) amino-acids 868-1130) of JAK1 and the human threonine/serine-specific kinase CDC2 (24) (amino acids 9-272) are aligned in order to maximize identity. The kinase-related domains have been divided into three segments and the number of amino acid residues separating each segment appears at the end of each line. Motifs held in common between at least two of these domains are both bolded and boxed. Roman numerals above the alignment correspond to the conserved domain nomenclature devised by Hanks et al (1988).
   Panel B. Hydropathy plot of the human JAK1 protein. The protein sequence of human JAK1 (including the 10 extra amino acids which precede the most likely initiation codon) were analysed by the hydrophilicity algorithm of Kyte and Doolittle (1982) using a - span length of 25 amino acids. The relative locations of the two kinase related domains are marked as Domain- 1 and PTK. The absence of a hydrophobic transmembrane domain is clearly seen, as can the presence of a highly hydrophilic region between amino acids 323 and 350.

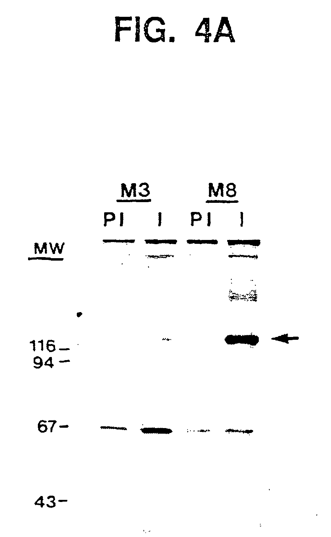

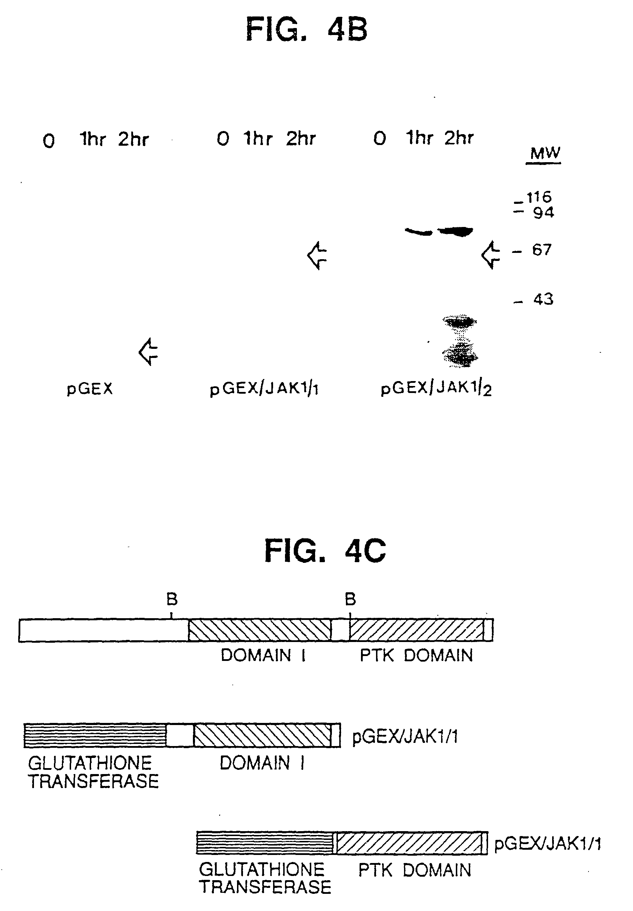

Figure 4 is a representation of an analysis of the JAK1 protein.
   Panel A. Cellular proteins of the murine mammary fibroblast cell line (17) were labelled with ³⁵S-methionine (panel A) and immunoprecipitated with either pre-immune (PI) or immune (I) anti-JAK rabbit antiserum (raised in rabbit M8 against the pGEX/JAK1/1 fusion protein or the C-terminal peptide [M3]) and fractionated on a 9.5% SDS-PAGE gel (Laemmli, 1970). Both rabbit antisera specifically immunopreciptated an ³⁵S-labelled protein of apparent molecular weight 130,000D.
   Panel B. Demonstration of tyrosine kinase activity in JAK1 bacterial fusion proteins. JAK1 fusion proteins were generated using pGEX2 (Smith and Johnson, 1988). The entire domain-1 region was included in construct pGEX/IAK1/1. The PTK domain portion of the fusion protein extended to the BamHI site 15 nucleotides 5' of the first glycine codon of the GXGXXG motif of the ATP binding site. An empty vector control was also performed. The bacteria were induced by the addition of 1mM IPTG as described by Smith and Johnson (1988) and two 1ml aliquots of the bacteria were removed at 60 minutes and 120 minutes post-induction and lysed with SDS sample buffer. Western analysis of the samples was performed using anti-phosphotyrosine antisera (PY-20 [ICN]). The arrow heads mark the positions of the GEX-JAK fusion proteins, in each induction.
   Panel C. Construction of the pGEX/JAK fusion proteins. The locations of the two kinase related domains of JAK1 are shown, and below, the structure of the fusion proteins with the glutathione S-transferase gene.

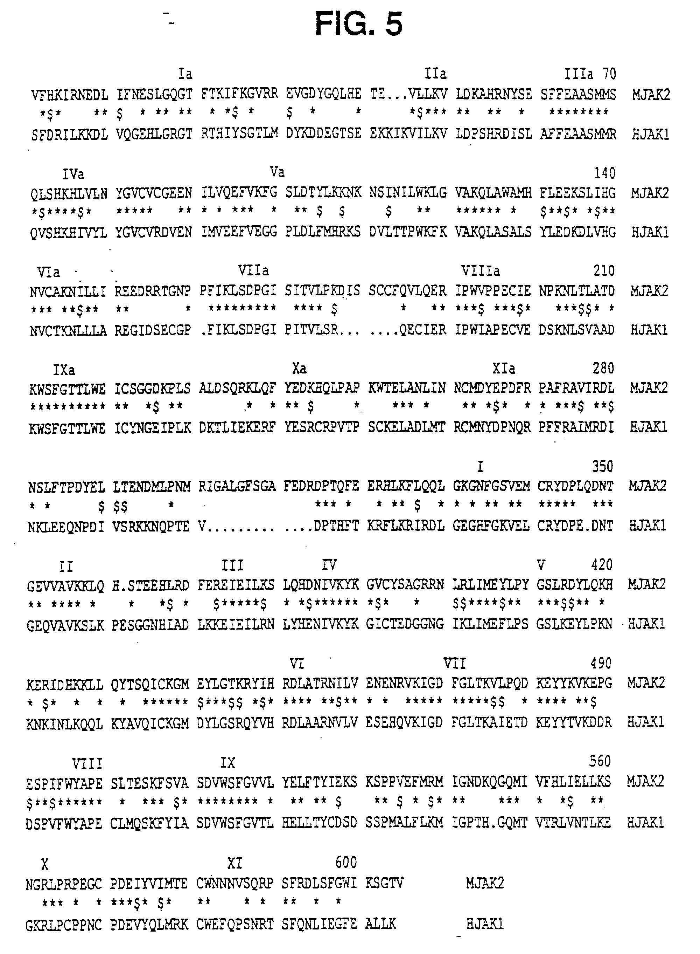

Figure 5 is a representation of a sequence comparison between JAK1 and JAK2 kinase-related domains. The deduced amino acid sequence of murine JAK2 was compared to the human JAK1 amino acid sequence by application of an alignment programme of the Staden VAX-based suite of Sequence analysis programmes. Asterisks (*) denote identity, dollar signs (S) denote conservative substitutions. Sequences are numbered with respect to the JAK1 sequence. The extent of the domain- 1 and PTK domains is shown by arrows above the amino acid sequence.

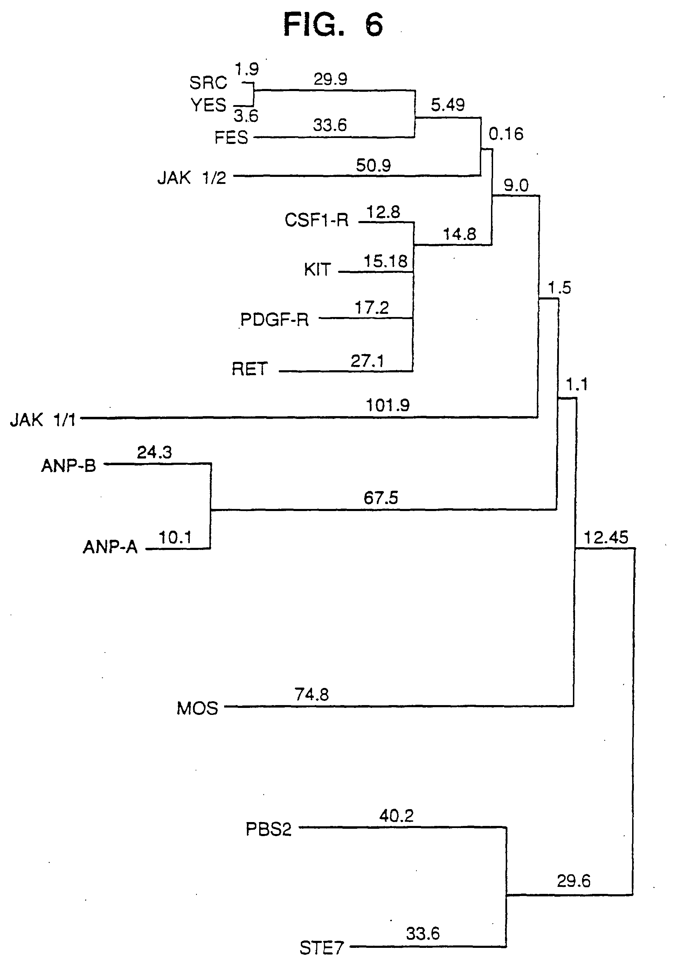

Figure 6 is a graphical representation of a phylogenetic analysis of the two JAK1 Kinase-like domains. The tree building concept of Fitch and Margoliash (1967) as implemented by Feng and Doolittle (1987) and Hanks et al (1988) was used to generate a phylogenetic tree as described in Example 1. In each case the catalytic domain alone was used for comparison. The two kinase related domains of the JAK1 protein were compared independently. Branch order is a function of structural similarity, branch length a function of sequence identity. The abbreviations used are: SRC= c-src; YES = c-Yes; FES = c-fes; CSFI-R = Colony stimulating factor- 1 receptor; KIT= c-kit; PDGF-R= Platelet derived growth factor receptor-A; RET= c-RET; ANP-A= Atrial naturetic peptide receptor-A; ANP-B = Atrial naturetic peptide receptor-B; MOS = c-mos; PBS2 = polyxin B antibiotic resistance gene product; STE7= sterile mutant wild-type allele gene product; JAK1/1= Domain-1 of Human JAK1; JAK1 /2= PTK domain of Human JAK1.

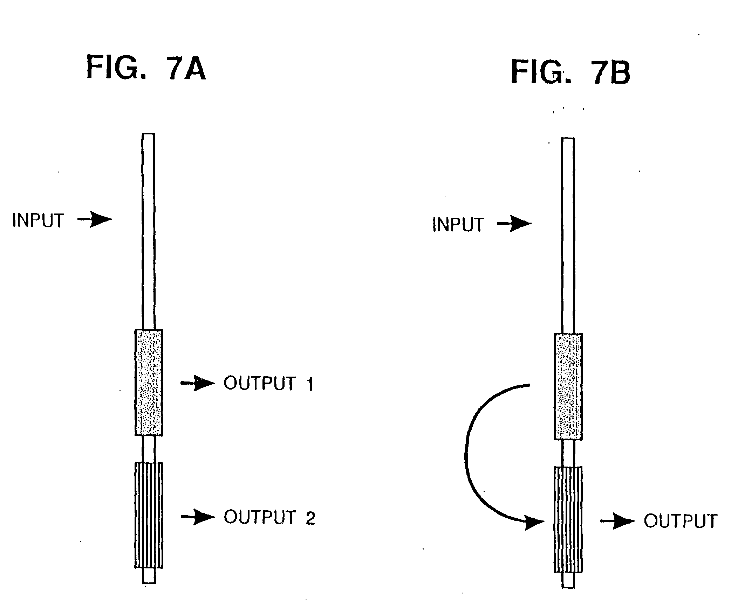

Figure 7 is a diagramatic representation showing models for the role of members of the JAK family of PTKs in signal transduction. Two possible scenarios are considered based on an extrapolation of the current notions of the role of PTKs in signal transduction. In panel A the N-terminal domain of the JAK protein serves to sense a particular metabolic cue and convert this input into two distinct outputs. Presumably the output of the second PTK-related domain is tyrosine kinase activity; the activity of Domain- 1 remains unknown. In panel B an alternative scenario is considered. In this case the function of Domain- 1 is the regulation of the PTK domain. In this scenario the sole output of the JAK protein is the PTK activity.
Figure 8 is a representation of a nucleotide sequence and predicted amino acid sequence of murine JAK2. The nucleotide sequence is numbered beneath each line of sequence, from the first nucleotide of the most 5' clone. The predicted amino acid sequence, in one letter code, is numbered at the end of each line of sequence. The two putative kinase domains are shown boxed with arrows, and the kinase consensus motifs are enumerated according to the nomenclature of Hanks et al (1988). The subscript a denotes the kinase-related motifs present in the first kinase-related domain, which are numbered according to the same nomenclature. (Sequence Id. 2)

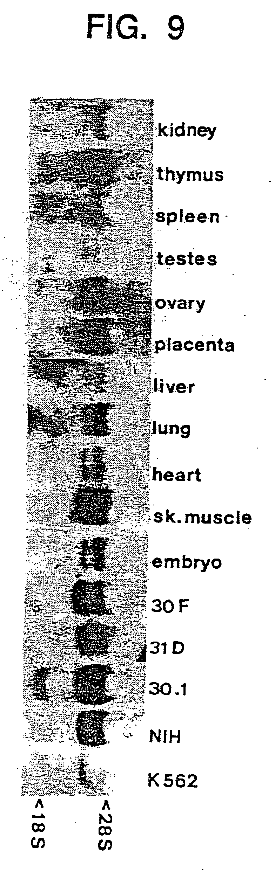

Figure 9 is a photographic representation showing expression of JAK2 in murine tissues. Northern blot analysis of 5 µg of mRNA from each of the tissues shown on top of the figure and from various murine (30F: mammary fibroblasts; 31A: mammary epithelial cells; 30.1: factor independent subline of the hemopoietic cell line FDC.P1; NIH: fibroblasts) and human (K562: chronic myelogenous leukaemic cells) cell line. The blots were hybridized with a ³²P-labelled 2.2 kb JAK2 probe and autoradiography was for 4 days. The relative mobilities of the 28S and the 18S rRNA are indicated.

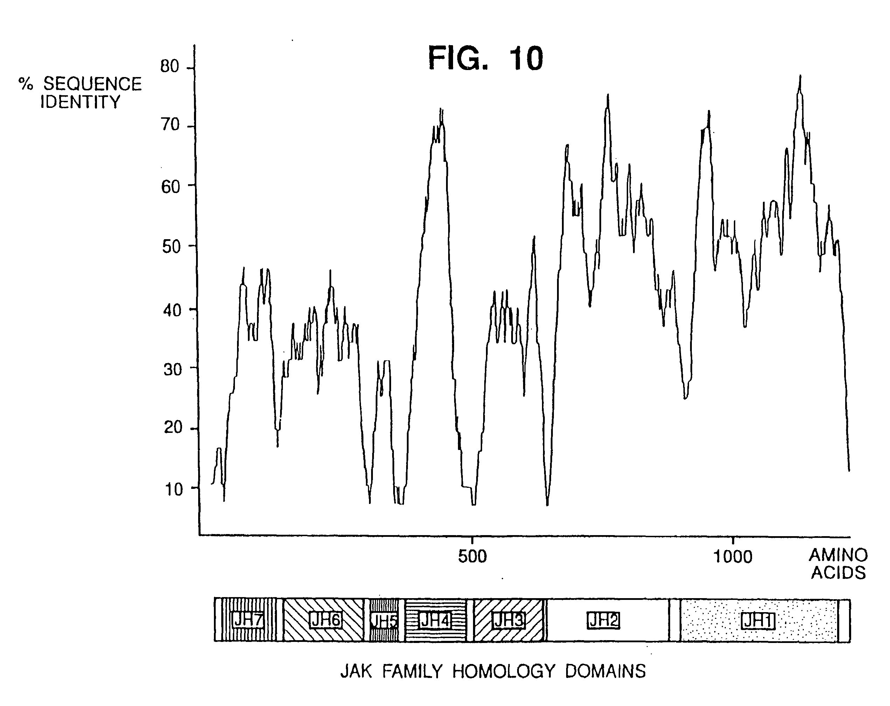

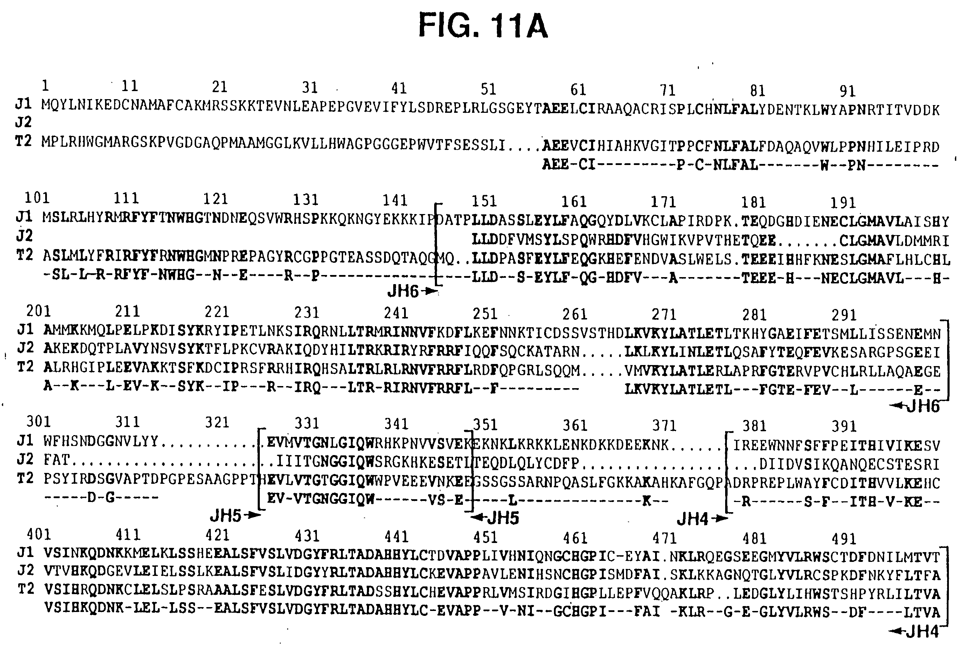

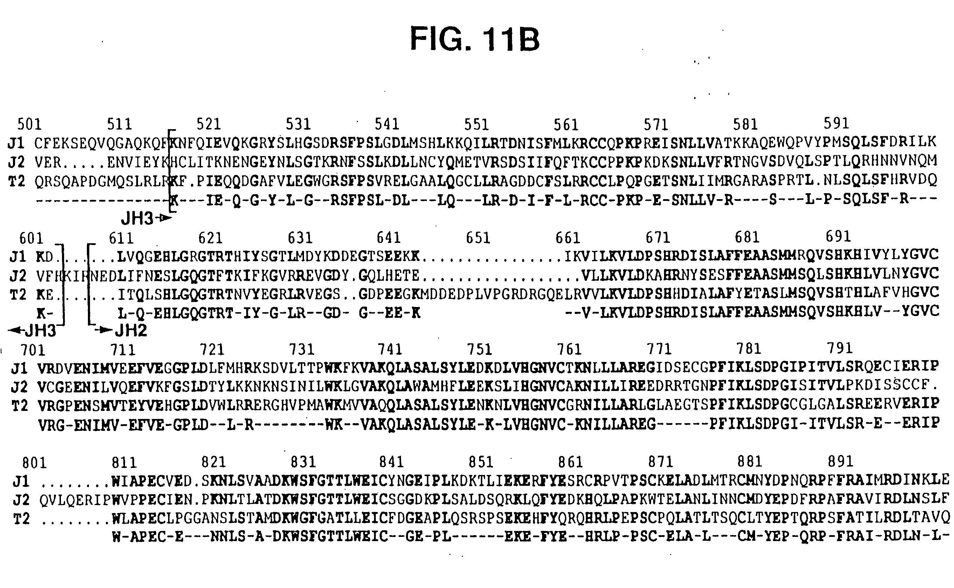

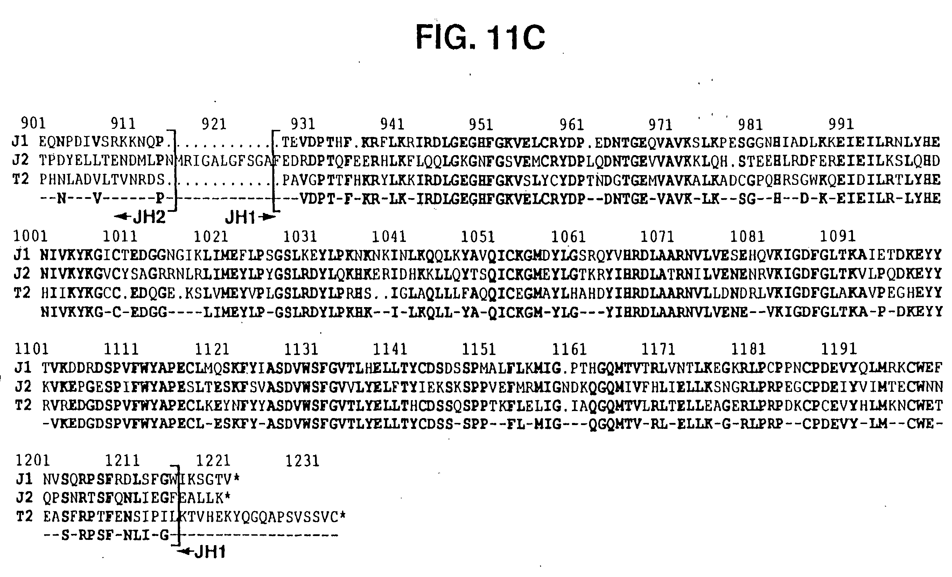

Figure 10 is a graphical representation showing comparison of JAK1 and TYK2 amino acid sequences. The amino acid sequences of JAK1 (Wilks et al, 1991) and TYK2 (Firmbach-Kraft et al, 1990) were compared using the HOMOLOGY option in the programme SEQMATCH, using a window length of 21 amino acids. The ordinate of the graph represent the percentage identiy between the two sequences, the abscissa represents the amino acid position in JAK1 at which the particular level of identity was calculated. The shaded boxes below the graph represent arbitrarily ascribed JAK homology domains as discussed in the text and further demonstrated in Figure 11.
Figure 11 is a representation showing amino acid sequence comparison of members of the JAK family of PTKs. The amino acid sequences of JAK1 (Wilks et al, 1991) (designated Jl in this figure), JAK2 (J2 in this figure), and TYK2 (Firmbach-Kraft et al, 1990) (T2 in this figure) were aligned using the CLUSTAL program (Higgins and Sharp, 1988). The numbering system is relative only to the first amino acid of JAK1, and does not take into account the insertion of gaps into this sequence, it is therefore useful only as a relative measure of location. The extent of each of the JAK homology domains was determined with reference to the homology plot shown in Figure 10. Amino acid positions conserved in at least 2 out of the 3 sequences presented are bolded and presented below the TYK2 sequence as a consensus sequence.

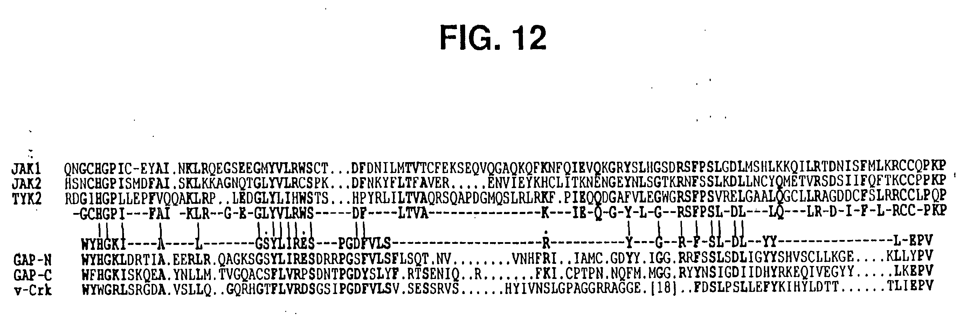

Figure 12 is a representation showing a comparison of the JH3/JH4 domain region with SH2 domains. The two SH2 domains of GAP (the more N-terminal domain denominated GAP-N (residues 178-269), the more C-terminal, GAP-C, (residues 348-438) (Trahey et al, 1988), and the SH2 domain of v-crk (residues 248-354) (Mayer et al, 1988) were compared with the JH3/JH4 of JAK1 (residues 425-536) (Wilks et al, 1991), JAK2 (residues 252-359) (this manuscript) and TYK2 (residues 449-555) (Firmbach-Kraft et al, 1990). Amino acids held in common between the two classes of sequence are denoted by vertical lines between the two sets of sequences. Conserved residues held in common by members of the same class of domain are bolded.

### EXAMPLE 1

### MATERIALS AND METHODS

### Screening of cDNA libraries

Several cDNA libraries were screened according to the protocols outlined in Maniatis et al, (1982). cDNA libraries from Murine NFS TPA activated spleen (Clontech cat.# ML1018), murine swiss-albino 3T3 fibroblast (Clontech cat.#. 1023b), murine balb/c bone marrow (Clontech cat.# ML1007), murine swiss-webster whole brain (Clontech cat.# ML1002), murine ICR linoleic. acid activated pleural macrophage (Clontech cat.# ML1005b), and human 1st-trimester foetal liver (Clontech cat.# HL1005b) were all generated in λgt 11. cDNA libraries from murine Balb/c testis (Clontech cat.# ML1020b), murine day 10 embryonic neuro-epithelium (Reid et al, 1990) and human foreskin fibroblast cell line AG1518 (Claesson-Welsh et al, 1989) were generated in λgtlO. Around 10⁶ recombinants of each of these libraries were screened on each occasion.

Library screening was carried out as follows. The FD22 (JAK1) PCR clone was labelled by nick-translation (Maniatis et al. 1982) and used to screen the murine libraries. A murine cDNA clone of 1.8kb was isolated amongst 3 other positives from the neuro-epithelial, and bone marrow cDNA libraries. Two full-length human JAK1 cDNA clones were isolated from the unamplified human foreskin fibroblast cell-line AG1518 by using the murine cDNA as a probe. Hybridisation was at 650C in 6xSSC; 1% SDS; 0.5% Blotto; 200 µg/ml sonicated and denatured herring sperm DNA. After hybridisation, the stringency of the final wash was 0.2xSSC; 0.1%SDS at 650C. Filters were autoradiographed overnight using Kodak XAR-5 X-ray film.

For JAK2, the murine macrophage was screened first with the FD 17 (JAK2) PCR clone, yielding 5 positives, and a portion of the longest cDNA clone isolated and used to screen the remaining cDNA libraries. Hybridisation conditions were as above for JAK1.

### DNA sequencing

Two strategies were employed for the sequencing of JAK1 and JAK2 cDNA clones. In the case of the human JAK1 sequence, the Erase-a-Base kit (PROMEGA) was employed to generate nested deletions of the largest EcoRI fragment. All of the murine JAK2 sequence data, and the remainder of the human JAK1 sequence, was determined using oligonucleotide primers based on previously determined DNA sequence. In each case the sequence information was generated using the dideoxynucleotide chain termination method (Sanger et al. 1977). All sequence information was determined on both strands.

### Northern Analysis

Poly A+ mRNA samples were prepared as elsewhere described elsewhere (Wilks and Kurban, 1988). Aliquots (1 µg) were analysed by electrophoresis on a 1% agarose gel containing 2.2M formaldehyde; 20mM MOPS,pH 6.8; 1mM EDTA; 5mM sodium acetate, and transferred to Hybond (Amersham, cat #RPN303N) or nitrocellulose (Schleicher & Schuell,BA85, cat #401196) membranes. Filters were prehybridised for 4hr in 50% formamide containing 3xSSC; 5xDenhardts; 10mM HEPES pH 7.0; 100µg.ml 1; poly C;100µg/ml denatured herring sperm DNA; 10µg/ml E. coli DNA; 0.1% SDS, and hybridised in the same solution with nick-translated ³²P-labelled murine or human JAK1 or JAK2 insert, for 18hr. at 42°C. Filters were washed to a final stringency of 0.2xSSC; 0.1% SDS at 65°C, before exposure to Kodak XAR-5 X-ray film, with two intensifying screens.

### Antibody Reagents and Protein Analysis

Polyclonal rabbit antisera M7 and M8 were raised against affinity purified pGEX/JAK1/l bacterial fusion protein (see section on kinase assays). Polyclonal antibodies M3 and M4 against the C-terminal peptide (-TSFQNLIECFEALLKC-) of JAK1 were raised in rabbits. Peptide was coupled to Keyhole Limpet Heamocyanin with 0.05% gluteraldehyde, emulsified in Freunds' complete adjuvant and injected intradermally at several sites. The animals were boosted four and seven weeks later with coupled peptide emulsified in Freunds' incomplete adjuvant and bled ten days after the last injection.

Cells were metabolically labelled with either ³⁵S-methionine or ³²P-orthophosphate in methionine- or phosphate-free medium containing 100µCi/ml and 1mCi/ml isotope respectively. RIPA-buffer (20mM Tris, pH7.5 containing 1% Triton X100, 1% Na deoxycholate, 0.1% SDS, 1mM EDTA, 1mM PMSF) extracts were incubated on ice with antiserum and immune-complexes isolated using Protein A bearing Staphylococus aureus bacteria. Proteins were resolved by SDS-PAGE (Laemmli, 1970) and radioactively labelled bands detected by exposure to X-ray film (Kodak XAR-5). The RIPA buffer for ³²P-labelled cells contained in addition 20mM EDTA, 10mM NaF, 100 µM orothovanadate as phosphatase inhibitors.

Phosphoamino-acid analysis of excised ³²P-labelled bands was carried out exactly as described by Hunter and Sefton (1980) Western blot analysis was performed as described by Towbin et al, (1979) as modified in Ziemiecki et al (1990) using either alkaline phosphatase or ¹²⁵I-labelled protein-A as a detection system.

### Protein Kinase Assays

A variety of protocols have been tried in order to reveal the PTK activity of the JAK1 protein. First, extraction of murine mammary fibroblasts, Reichmann et al (1989) has been performed in a range of buffers, containing Triton-X100 or Nonidet P40 (1.0%) alone, or with added Sodium Deoxycholate (0.5% or 1.0%) or in RIPA buffer (containing 1.0% Triton-X100. 1.0% Sodium Deoxycholate; 0.1% Sodium Dodecylsulphate). Cells have been extracted in the presence or absence of phosphatase inhibitors, such as 20mM EDTA, 10mM NaF and 100µM Na2V04.

After immunoprecipitation, kinase assays have been performed in a range of ATP concentrations (100nM-10mM) or with carrier-free γ-32P-ATP (Amersham cat #10169) in either 20mM Tris, pH 7.4 or 50mMM HEPES pH 7.4, with either 10mM Mn⁺⁺, Mg⁺⁺ or Zn⁺⁺ as divalent cation. Incubations have been performed on ice (15 min), at 25°C (15 min), at 30°C (15 min) or at 37°C (2 min) in the presence or absence of the phosphatase inhibitor Na2V04. Finally, γ-32P-GTP was employed as phosphate donor in lieu of γ-32P-ATP, with no success.

In order to generate the JAK1/glutathione transferase fusion proteins shown in Figure 4, domain-1 (from nucleotides 1770-2672 in Fig.2) and the PTK domain (from nucleotides 2672-end in Fig 2, thus including 5 extra amino acids beyond the ATP binding glycine motif) were each fused into the BamHI site of pGEX2. The fusion protein was induced by the addition of 1mM IPTG as described elsewhere (Smith and Johnson, 1983) and Western blot analysis performed on an induction time course with the M3 anti-JAK1 serum, and the anti-phosphotyrosinc antiserum (Kamps and Sefton, 1988). Several sources of anti-phosphotyrosine antisera were tried. The data in Figure 4b were obtained using a commercially available monoclonal antibody preparation PY-20 (ICN). In control experiments, induction of the insert-less pGEX or pGEX/JAK1 fusion protein produced no detectable tyrosine phosphorylation of bacterial substrates and the reactivity of the anti-phosphotyrosine antiserum could be completely abolished by the additional of phenyl phosphate.

### Computer Aided Sequence Analysis

Amino acid sequence comparisons were performed using an alignment programme from the Staden-based suite of programmes on a VAX VMS 5.2. The phylogenetic analysis of the two kinase-like domains of JAK1 was performed using the tree-building concept of Fitch and Margoliash (1967) as implemented by Feng and Doolittle (1987). The SCORE programme used to construct the difference matrices from which the trees were derived using the BORD and BLEN programmes, were all the gift of Dr R Doolittle of the University of California - San Diego.

The sequence alignment shown in Figure 11 was assembled using the CLUSTRAL program (Higgins and Sharp, 1988) on a VAX VMS 5.2 minocomputer. The homology plot shown in Figure 10 was assembled using the HOMOLOGY option of the programme SEQMATCH. Database searches with each of the JAK homolgoy domains was reformed using the FASTA programme, based on the Pearson/Lippman algorithm (Pearson and Lippman, 1988).

### RACE/Anchor PCR

RACE/Anchor PCR (Frohman et al., 1990, Loh et al., 1990) was performed by a modification of the original protocol. Briefly, 2µg of poly(A+) mRNA is converted to cDNA using an Amersham cDNA synthesis kit (cat No. RPN 1256) and 40 ng. of a JAK2 specific oligonucleotide primer (5'-TACACCTTTAAATATTTTTGT-3'). Prior to the addition of the reverse transcriptase, the reaction mixture was heated to 65°C. cDNA synthesis was inititated by the addition of 20 units of reverse transcriptase, and the reaction incubated at 55°C for 75 minutes. The newly sunthesised cDNA was recovered by passage through a spun sephadex column (Maniatis et al., 1982) followed by ethanol precipitation. The mRNA/cDNA heteroduplex was G-Tailed in 30µl containing 140 mM potassium cacodylate, 30 mM Tris, (pH7.2), 1mM CaCl₂, 0.1mM DTT, 6mM dGTP and 15 units of TdT (IBI), for 10 minutes at 37°C. The reaction was terminated by heating to 65°C for 15 minutes and then diluted to 500 µl with 10mM Tris. HCl (pH7.5). 1mM EDTA. For the RACE/Anchor PCR, 10µl of the tailed cDNA was reconstituted into 100µl PCR buffer (50mM KCl, 10mM Tris. HCl[pH8.3], 1.5mM MgCl₂, 0.01% gelatin, 200 µM of each dNTP) to this was added 50ng of "poly-C" oligonucleotide primer (5'-CTCGAGTCGACGAATTC₁₄-3') and 2.5 units of TAQ polymerase (Cetus). The complementary strand of the cDNA was synthesised with one cycle of 95°C (5 minutes), 52°C (5 minutes) and 68°C (40 minutes), whereupon 500 ng of the "RACE/Anchor" primer (5'-CTCGAGTCGACGAATTC-3') and a nested JAK2 specific primer (5'-CTTGCTTAATACTGACATCA-3') were added and the reaction mix subjected to 30 cycles of 95°C (1 minute), 52°C (2 minutes) and 68°C (5 minutes). The PCR product was phenol/chloroform extracted, precipitated and resuspended in 100µl of water. The amplified material was then kinased, size fractionated on a low-melting temperature agarose gel and cloned into SmaI cleaved M13mp8. Plaques were screened by hybridisation with a JAK2 cDNA and positives sequenced.

### EXAMPLE 2

### Isolation and DNA sequencing of cDNA clones encoding JAK1

JAK1 cDNA was cloned using PCR. Northern analysis (Figure 1a and b) demonstrated that in both mouse and human tissues and cell lines FD22 (JAK1) was encoded by a single widely expressed 5.4kb mRNA. Human cDNA clones of FD22 (JAK1) were isolated from a human foreskin fibroblast cell line (AG 1518) cDNA library (Claesson-Welsh et al, 1989). Two of the 8 primary isolates cloned contained inserts which were candidates for being full-length cDNAs ( - 5.3kb).

The nucleotide sequence of human JAK1 is shown in Figure 2. The 5' end of the clone has stop codons in all 3 reading frames prior to the putative initiation ATG. Two ATG start codons in frame with the longest open reading frame were found at positions 40 and 76 in the nucleotide sequence shown in Figure 2. The first of these is embedded in a particularly poor "Kozak" consensus sequence (Kozak, 1984) (-TAAATGCAG-), whereas the second matches strongly with the optimal consensus sequence defined by Kozak, namely -GCCATGGCT-. The second ATG is considered to be the initiation codon for this protein, since the first one transgresses one of the strongest correlations found in the sequences which precede initiation codons, namely the presence of a T residue (in lieu of an A residue) 3 nucleotides-before the ATG sequence. At the 3'end, an in-frame stop codon at position 3502 defines the C-terminus of the protein. A large (1.405 kb) 3' untranslated region containing a polyadenylation signal completes the mRNA sequence.

The JAK1 coding region of 3426bp encodes a protein of 1142 amino-acids with a calculated molecular mass of 132,000 daltons. The PTK catalytic comain is located towards the C-terminus of the JAK1 protein (Figure 2). In describing the structural features of this domain we have chosen to adopt the nomenclature of Hanks et al (1988). The putative ATP binding site composed of the motif GLY-X-GLY-X-X-GLY- (subdomain 1) followed by an invariant lysine residue (subdomain II) is located between amino acid residues 871 and 896 of the JAK1 protein. The core motifs of the PTK catalytic domain (sub-domains VI to IX) are also in their appropriate locations, and are well conserved with respect to their primary sequence and their relationship to each other. The presence of a tyrosine residue at position 1022 in the JAK1 protein, 11 residues C-terminal to sub-domain VII (a similarly placed tyrosine is a site of tyrosine autophosphorylation in v-fps; Weinmaster et al. 1984) is a consistent feature of members of the PTK family and is considered diagnostic of membership of this class of kinases. The arginine residue at position 1126 (domain XI) marks the end of the highly conserved regions of the PTK catalytic domain and the entire catalytic domain of 255 amino acids is approximately 28% (with c-fes: Wilks and Kurbon, 1988) to 37% (with TRK; Kozman et al, 1988) identical to other functionally defined PTKs. Finally, there is a rare variant of the highly conserved subdomain VIII motif (residues 1032-1039), which is believed to lie close to the active site (Hanks et al, 1988). The presence of phenylalanine and tyrosine flanking the conserved tryptophan in this motif is unique to JAK1 and JAK2.

A second protein kinase-related domain (designated here Domain-1) is located between amino acids 578 and 824, 47 amino acids N-teriminal to the putative PTK domain. All of the conserved elements of protein kinases are preserved spatially in this domain. In Figure 2 these elements are numbered with respect to their similarity to the subdomains of protein kinases described by Hanks et al, (1988) (with the suffixₐ, e.g. IIIₐ) and the amino acid sequences of the two kinases-related domains of JAK1 are compared to each other and to human CDC2 (Lee and Nurse, 1987) in Figure 3a. The overall structural similarity of this domain to the kinase domains of both the PTK and threonine/serine kinase families strongly suggest that this region of the protein also functions as a protein kinase. There are, however, significant differences in the sequences of key motifs within this domain which suggest that Domain-1 may confer a catalytic activity other than serine/threonine or tyrosine phosphorylation. For example, sub-domain VIₐ is poorly conserved with respect to the equivalent motifs in the other kinase families, and the normally invariant -ASP-PHE-GLY- sequence of the PTK and threonine/serine kinase families (sub-domain VIIₐ) is replaced by the motif ASP-PRO-GLY- in Domain-1 of JAK1. As has been noted elsewhere, the conservation of the precise sequence of sub-domain VI in the PTK and threonine/serin kinase families appears to correlate with the substrate specificity of the kinase. Thus, it is possible that Domain-1 of the JAK1 kinase has a substrate specificity other than that exhibited by the PTK and threonine/serine kinase has a substrate specificity other than that exhibited by the PTK and threonine/serine kinases. In support of this notion there are subtle differences in the normally consistent spacing between certain key motifs in Domain-1 of JAK1. The components of the ATP binding site (sub-domains Iₐ and IIₐ) are some 7 amino acids further apart in this domain that they are in both the PTK family and the threonine/serine kinase family. Moreover, the spacing between sub-domains VIₐ and VIIₐ in this region is also longer by 9 amino acids. Conversely, the distance between sub-domains VIIₐ and IXₐ is 7 amino acids shorter than the corresponding region in the PTK catalytic domain. The overall structure of this domain can be expected to be somewhat different to the catalytic domains of the members of the PTK and threorzine/serine kinase families.

The sequences N-terminal to Domain-1 bear no homology to any other portion of a previously described protein kinase. Specifically, no homology was detected to the SH2 domain described for the cytoplasmic PTKs such as c-fes/fps. (Sadowski et al, 1986) GAP (Trahey et al, 1988) and the phospholipase-C family of proteins (Suh et al, 1988). This is a particularly interesting observation since no other non-receptor PTK has been described which lacks this feature. A hydrophilicity plot failed to demonstrate the present of a hydrophobic domain characteristic of the growth factor receptor type of PTK (Figure 3b) suggesting that this protein is wholly intracellular like other members of the non-receptor class of PTKs. The one outstanding feature of the JAK1 hydropathy plot is the highly hydrophilic sequence between residues 320-350. This sequence is not conserved in the murine JAK2 protein, however, its remarkable nature suggests that. it may well be involved in some function of the JAK1 protein.

### Expression of JAK1 protein

Several antisera were geneated against the human JAK1 protein. Polyclonal antisera directed against the hexadecamer -TSFQNLIECFEALLKC- (the C-terminal 15 amino acids of JAK1) were raised in rabbits and used to investigate the nature of the JAK1 protein. A second rabbit antiserum was generated using a pGEX bacterial fusion protein containing the entire Domain-1 region of the human JAK1 protein (see Example 1). Preliminary sequence analysis of cDNA clones of murine JAK1 demonstrated that the C-terminus of the human and murine versions of this protein were identical whereas the murine and human Domain-1 regions exhibited a very high degree of identify. Both systems have thus been used interchangably in the investigation of the properties of the JAK1 protein.

Both antisera have been used for Western blot analyses and immunoprecipitation studies and the data confirm the mRNA expression studies shown in Figure 1. For example, antisera M3 and M8 both immunoprecipitate a protein of the same apparent molecular weight (130 kDaltons) from ³⁵S-methionine labelled murine breast fibroblasts (Fig. 4a). From the same source, ³²P-orthophosphate labelled JAK1 was immunoprecipitated as a phosphothreonine and phosphoserine containing phosphoprotein. It is a feature characteristic of members of the protein tyrosine kinase family that they are able to accomplish an act of self phosphorylation in vitro. Intriguingly, despite the high degree of sequence similarity held by the PTK-related sequence of JAK1 to the PTK family in general, it was not possible to demonstrate tyrosine kinase catalytic activity in immunoprecipitates of this protein from any of the murine or human sources tested. A wide range of possibilities has been tested in search of suitable conditions for the demonstration of this activity. These are listed in Example 1. The reason for the lack of activity may lie with a steric effect of the antibody in the active site of the enzyme.

In order to determine whether domain-1 or the PTK domain, in isolation, bore catalytic activity, bacterial fusion proteins of each were generated with the glutathione transferase protein of Schistosoma-japonicum (Smith and Johnson, 1988) and an attempt was made to demonstrate with the aid of anti-phosphotyrosine antibodies (Kamps and Sefton, 1988) the co-ordinate induction of the fusion protein and tyrosine phosphorylated protein. In this system there is no cross-reactive background of the anti-phosphotyrsine antiserum, since there are no tyrosine kinases in bacteria (Fig. 4b). The phosphorylation of bacterial proteins on tyrosine is thus easily detectable with such a serum. In this series of experiments neither pGEX without insert nor pGEX bearing Domain-1 (pGEX/JAK/1/1) demonstrated any tyrosine kinase activity. The pGEX/JAK/1 fusion protein was further purified by affinity chromatography on a reduced glutathione column and have failed to detect any kinase activity using either histones, casein or enolase as an exogenous substrate. The pattern of inducible tyrosine phosphorylation exhibited by the pGEX PTK fusion protein (pGEX/JAK/2) (Fig. 4b) is ususually simple for an ectopically expressed PTK fusion protein. Remarkably, the autophosphorylation of the fusion protein itself does not seem to occur, an observation which may go some way toward explaining why we have had difficulty in demonstrating PTK activity in the intact protein.

cDNA clones covering the coding region of the PCR clone FD17 (JAK2) have been isolated from a range of murine cDNA libraries. The predicted amino acid sequences of JAK2 and JAK1 show several regions of significant similarity to each other (Fig. 5, see also Example 3).

### Phylogenetic analysis

The phylogenetic relationship of the catalytic domains of most of the protein kinases has been determined using the tree-building programme of Feng and Doolittle (1987). Figure 6 shows the phylogenetic relationship of the two kinase-related domains of the JAK1 protein to the rest of the kinase family. It is concluded from this family tree that these two domains had a common ancestor which pre-dated the development of the PTK sub-family. It is of interest to note that the kinase-related domains of the ANP-receptor/guanylate cyclase family diverge at a point close by.

### EXAMPLE 3

### Cloning and sequencing of JAK2

### Sequence of Murine JAK2

The PCR clone FD17 was used as a basis to begin the cloning of longer cDNA clones of murine JAK2, cDNAs were isolated from a range of cDNA libraries, and by RACE (Frohman et al, 1989, Loh et al, 1989). The sequence of murine JAK2 is presented in Figure 8. The predicted amino acid sequence indicates that this protein is highly related to JAK1. At the C-terminus, and extending approximately 270 amino acids towards the N-terminus (AA 715-980), are sequences bearing all the hall marks of a PTK catalytic domain. These are labelled in Figure 8 according to the Hanks nomenclature. Immediately N-terminal to this (AA 400-660) lies the kinase-related domain characteristic of this class of PTKs (Wilks et al, 1991). The approach outlined in Example 2 in relation to JAK1 was followed and assigned these kinase related domains according to the Hanks nomenclature, appending the suffix Na to denote their origin. One unusual feature of this domain is an apparent insertion of seven amino acids between elements VIIa and VIIIa (Hanks nomenclature; Hanks and Quinn, 1991) with respect to other members of this family. This feature appeared in only one clone of the four sequenced which covered this region, and it remains possible that its presence is due to an infrequent splicing abberation, rather than being of functional significance.

### Distribution of JAK2

Northern analysis of the expression of JAK2 in the mouse demonstrated two mRNA transcripts (4.8 and 4.4 kb) hybridizing to the JAK2 probe under low and high stringency hybridization conditions (Figure 9). It is intriguing to note that the levels of these transcripts alter with respect to one another in different tissues. For example, the kidney, spleen and lung appear to express predominantly the larger form, whereas ovary, placenta, skeletal (sk) muscle and all murine cell lines analyzed express both forms at about equal levels.

Under low stringency hybridization conditions the murine JAK2 probe recognizes human JAK2 RNA (K562), however, only the smaller transcript of 4.4 kb could be detected. At this point, the origins of either of the two transcripts are unclear and no differential splicing events which could account for the differences between them could be detected. However, the major source of size differential in these transcripts may lie in the use of different poly-adenylation signals. JAK2 is widely expressed in mouse organs, albeit to different levels. High expression was found in thymus, skeletal muscle, ovary and placenta, but JAK2 transcripts were barely detectable in testes or liver. In addition, JAK2 expression was detected in murine cell lines of fibroblastic (30F, NIH), epithelial (31D) and hemopoietic (30.1) origin.

### JAK Family Homology Domains.

The cloning of JAK1 and JAK2 has facilitated the identification of JAK family homology domains. Fig 10 shows a comparison of the amino acid sequences of JAK1. Sequence identity between these two proteins manifests itself as seven clearly defined homology domains. These seven domains are defined at a primary sequence level in Figure 11. The PTK domain is classified as the JAK-homology Domain 1 (JH1), the second kinase related domain as the JH2 Domain, and so on to JH7. The boundaries of the JAK homology domains are arbitrary, and may or may not define functional domains. However, their delineation is a useful device to aid the consideration of the overall structural similarity of this class of proteins. The structure of the JH1 and JH2 Domains are described in Example 2. The JH3 is one of the least highly conserved of the JAK homology domains, each family member bearing between 35% (JAK2) to 50% (JAK1) of the deduced consensus sequence. The JH4 domain bears the sequence -GLYVLRWS- close to its C-terminal boundary, which has some degree of homology to the SH2 domain core sequence (see below). In addition, the most highly conserved sub-domain of this region bears a potential tyrosine phosphorylation site, namely, -VDGYFRI-. Overall, the JH4 domain has between 51% (JAK2) and 64% (JAK1) of the deduced consensus sequence for this domain. Each of the remaining JAK homology domains has been independently screened against the N-BRL and EMBL databases using the FASTA programme. There were no compelling homologies found with anything in these databases. It is concluded that these domains are structurally and functionally conserved in members of the JAK family of PTKs, but may not, in contradistinction to the SH2 and SH3 domains of the src family of PTKs, have a role to play in other signal transduction molecules.

The apparent absence of an SH2 domain in any of the JAK family of PTKs is intriguing. Subtle sequence similarities have been detected between SH2 consensus sequences and portions of the JH3 and JH4 domains (H. Hanafusa and A. Bemards, personal communication). Fig 12 shows an alignment of these two domains. Whilst the similarity of the JH3 domain to SH2 domains is most evident in the region surrounding the SH2 core sequence (FLVRES), the homology does not extend far in either direction beyond this region, and only reappears again close to the C-terminal boundary of the SH2 domain. This lack of extensive homology, particularly in many of those elements most highly conserved between SH2 domains (Koch et al, 1991) (presumably indicating those residues most intimately involved in the conserved function of this domain), suggests that the homology detected is either happenstance, or the product of considerable sequence divergence in evolution. The SH2 domain is currently believed to interact with phosphorylated tyrosine residues on the substrates of PTKs (reviewed in Pawson, 1989; Koch et al, 1991). Whether the JH3/JH4 domains play a similar functional role remains to be determined.

### EXAMPLE 4

To show that JAKs are represented in a range of animals, oligonucleotide probes were prepared and used to amplify and screen genomes from a variety of animals. JAK DNA was detected in Drosophila, xenopus, mouse and human genomes. The main conserved sequence was DPG common to all animals tested.

### REFERENCES:

Claesson-Welsh, L., Eriksson, A, Westermark, B. and Heldin, C.H., Proc.Nat. Acad. Sci. USA 86: 4917-4921, 1989.
Feng, D.F. and Doolittle, R.F. Jour Mol. Evolution 25: 351-360, 1987.
Fitch, W.M. and Margoliash, E., Science 12: 279-284, 1967.
Hunter, T., and Sefton, B.M. Proc. Nat. Acad. Sci. 77: 1311-1315, 1980.
Kamps, M.P., and Sefton, B.M. Oncogene 2: 305-315,1988.
Kozak, M. Nucleic Acids Res. 12: 857-872,1984.
Kozma, S.C. Redmond, S.M.S., Xiano-Chang, F., Saurer, S.M. Groner, B., and Hynes, N.E. EMBO J. 7: 147-154,1988.
Kyte, J. and Doolittle, R.F. J. Mol. Biol 157: 105-132, 1982.
Laemmli, U.K. Nature (London) 227: 680-685,1970.
Lee, M.G. and Nurse, P. Nature (London) 327: 31-35,1987.
Maniatis, T, Fritsch, E.F., and Sambrook, J., in Molecular Cloning: A Laboratory Manual Cold Spring Harbor, N.Y. 1982.
Moran, MF., Koch, C-A., Sadowski, I., and Pawson, T. Oncogene 3: 665-672, 1988.
Reichmann, E., Ball, R., Groner, B., and Friis, R.R. J. Cell Biol 108: 1127-1138,1989.
Smith, D.B. and Johnson, K.S. Gene 67: 31-40, 1988.
Suh, P., Ryu, S.H., Moon, K.H., Suh, H.W., and Rhee, S.G. Cell 54: 161-169, 1988.
Towbin, H., Stehelin, T., and Gordon, J., Proc. Nat. Acad. Sci. USA 76: 4350-4354,1979.
Weinmaster, G., Zoller, M.M., Smith, M., Hinze, E., and Pawson, T. Cell 37: 559-563,1984.
Wilks, A.F. and Kurban, R.R. Oncogene 3: 289-294, 1988.
Ziemiecki, A, Mueller, R.G., Xiao-Chang, F, Hynes, N.E. and Kozma, S., EMBO J. 9: 191-196,1990.
Dymecki, S.M., Neiderhuber, J.E., and Desiderio, S.v. Science 247: 332-336, 1990.
Firmbach-Kraft, I., Byers, M., Showes, T., Dalla-Favera, R., and Krolewski, JJ., Oncogene 5: 1329-1336, 1990.
Frohman, MA, Dush, M.K. and Martin, G., Proc. Nat. Acad. Sci. USA 85: 8998-9002, 1988.
Hanks, S.K. and Quinn, A-M. Methods in Enzymology 200: 38-62, 1991.
Hanks, S.K., Quinn, A,M. and Hunter, T. Science 241: 42-52, 1988.
Higgins, D.G. and Sharp, P.M. Gene 73: 237-244, 1988.
Holtzman, D.A., Cook, W.D. and Dunn, A.R. Proc. Natl. Acad. Sci. USA 84: 8325-8329, 1987.
Koch, C.A., Anderson, D., Moran, M.F., Ellis, C., and Pawson, T., 252: 668-674,1991.
Loh, E.Y., Elliott, J.f., Cwirla, S., Lanier, LL. and Davis, M.M. Science 243: 217-220, 1989.
Marth, J.D., Peer, R., Krebs, E.G., and Perimutter, R.M. Cell 43: 393-404, 1985.
Martinez, R., Mathey-Prevot, B., Bernards, A. and Baltimore, D. Science 237: 411-414,1987.
Mayer, BJ., Hamaguchi, H., and Hanafusa, H., Nature 332: 272-274,1988.
Nishizawa, M., Semba, K., Yoshida, M.C. Yamamoto, T., Sasaki, M., and Toyoshima, K. Mol. Cell. Biol. 6: 511-517,1986.
Pawson, T., Oncogene 3: 491-495, 1988.
Pearson, W.R. and Lippman, DJ. Proc. Nat. Acad. Sci. 85: 2444-2448,1988.
Reid, H.H., Wilks, AF., and Bernard, O., Proc Nat. Acad. Sci 87: 1596-1600, 1990.
Sadowski, I., Stone, J.C., and Pawson, T. Mol Cell. Biol 6: 4396-4408, 1986.
Sanger, F., Nicklen, S., and Couson, A.R., Proc. Nat. Acad. Sci. USA 74: 5463-5467,1977.
Semba, K., Nishizawa, M., Myajima, N., Yoshida, M.C., Sukagawa, J., Yamanishi, Y., Sasaki, M., Yamamoto, T., and Toyoshima, K., Proc. Natl. Acad. Sci. 83: 5459-5463,1986.
Sukegawa, J., Semba, K., Yamanashi, Y., Nishizawa, M., Myajima, N., Kamamoto, T., and Toyoshima, K., Mol. Cell. Biol. 7: 41-47,1987.
Trahey, M., Wong, G., Halenbeck, R., Rubinfeld, B., Martin, G.A., Ladner, M., Long, C.M., Crosier, WJ., Watt, K., Koths, K., and McCormick, F., Science 243; 1697-1700,1988.
Wilks, A.F., Process in Growth Factor Reseach 2: 97-111, 1990.
Wilks, A.F., Harpur, A., Kurban, R.R., Ralph, SJ., Zuercher, G., and Ziemiecki, A. Molecular and Cellular Biology 11: 2057-2065,1991.
Yamamishi, Y., Fukushige, S.I., Semba, K., Sukegawa, J., Miyajima, N., Matsubara, K.I., Yamamoto, T., and toyoshima, K, Molec Cell Biol 7: 237-243,1987.

## Claims

1. A human or mouse protein tyrosine kinase-like (PTK) polypeptide comprising two protein kinase catalytic domains but no SH2 domain and (a) having the amino acid sequence of Figure 2 (JAK1) or (b) having an amino acid sequence which is encoded by a polynucleotide which hybridises to the complement of the polynucleotide sequence of Figure 2 under the stringency conditions of 6xSSC, 1% SDS, 0.5% Blotto 200µg /ml sonicated and denaturated herring sperm DNA at 65°C and wash at 0.2xSSC, 0.1% SDS at 65°C.

2. A human or mouse protein tyrosine kinase-like (PTK) polypeptide comprising two protein kinase catalytic domains but no SH2 domain and (a) having the amino acid sequence of Figure 8 (JAK2) or (b) having an amino acid sequence which is encoded by a polynucleotide which hybridises to the complement of the polynucleotide sequence of Figure 8 under the stringency conditions of 6xSSC, 1 % SDS, 0.5% Blotto 200µg /ml sonicated and denaturated herring sperm DNA at 65°C and wash at 0.2xSSC, 0.1% SDS at 65°C.

3. A polypeptide comprising an amino acid sequence selected from residues 576 to 825 of Figure 2, residues 868 to 1130 of Figure 2, residues 715 to 980 of Figure 8 or residues 400 to 660 of Figure 8.

4. A vector comprising a nucleotide sequence encoding a polypeptide according to any one of the preceding claims.

5. A nucleic acid molecule having the nucleotide sequence shown in Figure 2.

6. A nucleic acid molecule having the nucleotide sequence shown in Figure 8.

7. An antibody which specifically binds to the polypeptide according to any one of claims 1 to 3.

8. The antibody according to claim 7 wherein the antibody is.a monoclonal antibody.

9. A method for phosphorylating a protein *in vitro* comprising contacting said protein with a phosphorylating amount of protein tyrosine kinase polypeptide accocding to any one of claims 1 to 3 for a time and under conditions sufficient for said first protein to be phosphorylated.

10. The method according to claim 9 wherein the molecular weight of the protein tyrosine kinase molecule is from about 100,000 to about 200,000 Daltons.

11. The method according to claim 10 wherein the molecular weight is from about 120,000 to about 150,000 Daltons.

## Patentansprüche

1. Menschliches oder aus der Maus stammendes Protein-Tyrosinkinase-ähnliches (PTK)-Polypeptid, das zwei katalytische Domänen der Proteinkinase, jedoch keine SH2-Domäne umfasst und das (a) die Aminosäuresequenz der Figur 2 (JAK1) aufweist oder (b) eine Aminosäuresequenz aufweist, die durch ein Polynucleotid kodiert wird, welches mit dem Komplement der Polynucleotidsequenz der Figur 2 unter den Stringenzbedingungen von 6x SSC, 1 % SDS, 0,5 % Blotto, 200 µg/ml beschallte und denaturierte Heringssperma-DNS, bei 65 °C und Waschen mit 0,2x SSC, 0,1 % SDS bei 65 °C hybridisiert.

2. Menschliches oder aus der Maus stammendes Protein-Tyrosinkinase-ähnliches (PTK)-Polypeptid, das zwei katalytische Domänen der Proteinkinase, jedoch keine SH2-Domäne umfasst und das (a) die Aminosäuresequenz der Figur 8 (JAK2) aufweist oder (b) eine Aminosäuresequenz aufweist, die durch ein Polynucleotid kodiert wird, welches mit dem Komplement der Polynucleotidsequenz der Figur 8 unter den Stringenzbedingungen von 6x SSC, 1 % SDS, 0,5 % Blotto, 200 µg/ml beschallte und denaturierte Heringssperma-DNS, bei 65 °C und Waschen mit 0,2x SSC, 0,1 % SDS bei 65 °C hybridisiert.

3. Polypeptid, das eine Aminosäuresequenz, ausgewählt aus den Resten 576 bis 825 der Figur 2, den Resten 868 bis 1130 der Figur 2, den Resten 715 bis 980 der Figur 8 oder den Resten 400 bis 660 der Figur 8, umfasst.

4. Vektor, der eine Nucleotidsequenz umfasst, die ein Polypeptid gemäß irgendeinem der vorhergehenden Ansprüche kodiert.

5. Nucleinsäuremolekül, das die Nucleotidsequenz aufweist, die in Figur 2 gezeigt ist.

6. Nucleinsäuremolekül, das die Nucleotidsequenz aufweist, die in Figur 8 gezeigt ist.

7. Antikörper, der spezifisch an das Polypeptid gemäß irgendeinem der Ansprüche 1 bis 3 bindet.

8. Antikörper nach Anspruch 7, worin der Antikörper ein monoklonaler Antikörper ist.

9. Verfahren zur Phosphorylierung eines Proteins *in vitro,* umfassend das in Kontakt Bringen dieses Proteins mit einer phosphorylierenden Menge des Protein-Tyrosinkinase-Polypeptids gemäß einem der Ansprüche 1 bis 3 für eine Zeit und unter Bedingungen, die für dieses erste Protein ausreichend sind, um phosphoryliert zu werden.

10. Verfahren nach Anspruch 9, worin das Molekulargewicht des Protein-Tyrosinkinase-Moleküls von ungefähr 100.000 bis ungefähr 200.000 Dalton beträgt.

11. Verfahren nach Anspruch 10, worin das Molekulargewicht von ungefähr 120.000 bis ungefähr 150.000 Dalton beträgt.

## Revendications

1. Polypeptide analogue à une protéine tyrosine kinase (PTK) humaine ou de souris comprenant deux domaines catalytiques de protéine kinase mais pas de domaine SH2 et (a) ayant la séquence d'acides aminés de la figure 2 (JAK1) ou (b) ayant une séquence d'acides aminés qui est codée par un polynucléotide qui s'hybride au complément de la séquence polynucléotidique de la figure 2 dans les conditions de stringence de SSC 6X, SDS 1 %, Blotto 0,5 %, ADN de sperme de hareng soniqué et dénaturé 200 µg/ml, à 65 °C et lavage avec du SSC 0,2X, SDS 0,1 % à 65 °C.

2. Polypeptide analogue à une protéine tyrosine kinase (PTK) humaine ou de souris comprenant deux domaines catalytiques de protéine kinase mais pas de domaine SH2 et (a) ayant la séquence d'acides aminés de la figure 8 (JAK2) ou (b) ayant une séquence d'acides aminés qui est codée par un polynucléotide qui s'hybride au complément de la séquence polynucléotidique de la figure 8 dans les conditions de stringence de SSC 6X, SDS 1 %, Blotto 0,5 %, ADN de sperme de hareng soniqué et dénaturé 200 µg/ml, à 65°C et lavage avec du SSC 0,2X, SDS 0,1 % à 65°C.

3. Polypeptide comprenant une séquence d'acides aminés choisie parmi les résidus 576 à 825 de la figure 2, les résidus 868 à 1130 de la figure 2, les résidus 715 à 980 de la figure 8 ou les résidus 400 à 660 de la figure 8.

4. Vecteur comprenant une séquence nucléotidique codant un polypeptide selon l'une quelconque des revendications précédentes.

5. Molécule d'acide nucléique ayant la séquence nucléotidique présentée à la figure 2.

6. Molécule d'acide nucléique ayant la séquence nucléotidique présentée à la figure 8.

7. Anticorps qui se lie spécifiquement au polypeptide selon l'une quelconque des revendications 1 à 3.

8. Anticorps selon la revendication 7, l'anticorps étant un anticorps monoclonal.

9. Procédé pour phosphoryler une protéine *in vitro* comprenant le fait de mettre en contact ladite protéine avec une quantité phosphorylante de polypeptide de protéine tyrosine kinase selon l'une quelconque des revendications 1 à 3 pendant une durée et dans des conditions suffisantes pour que ladite première protéine soit phosphorylée.

10. Procédé selon la revendication 9, dans lequel la masse moléculaire de la molécule de protéine tyrosine kinase est d'environ 100 000 à environ 200 000 daltons.

11. Procédé selon la revendication 10, dans lequel la masse moléculaire est d'environ 120 000 à environ 150 000 daltons.
